(19) Europäisches Patentamt
European Patent Office
Office européen des brevets

(11) **EP 3 226 970 B1**

## (12) EUROPÄISCHE PATENTSCHRIFT

(45) Veröffentlichungstag und Bekanntmachung des Hinweises auf die Patenterteilung:
**03.01.2024 Patentblatt 2024/01**

(21) Anmeldenummer: **15802095.8**

(22) Anmeldetag: **30.11.2015**

(51) Internationale Patentklassifikation (IPC):
**A61N 1/372** (2006.01)   **A61N 1/378** (2006.01)
**A61N 1/36** (2006.01)   **H02M 3/335** (2006.01)
**H02M 3/00** (2006.01)   **H02J 50/12** (2016.01)
**H03J 3/02** (2006.01)   H02M 7/48 (2007.01)
**H02M 1/00** (2006.01)

(52) Gemeinsame Patentklassifikation (CPC):
**H02J 50/12; A61N 1/36125; A61N 1/37223; A61N 1/3787; H02M 3/01; H02M 3/015; H02M 3/33571; H03J 3/02;** H02J 2310/23; H02M 1/0058; H02M 7/4818; Y02B 70/10

(86) Internationale Anmeldenummer:
**PCT/EP2015/078104**

(87) Internationale Veröffentlichungsnummer:
**WO 2016/087391 (09.06.2016 Gazette 2016/23)**

(54) **REGELSCHALTUNG FÜR EINE BASISSTATION ZUM ÜBERTRAGEN EINER ENERGIE AUF EINEN EMPFÄNGER MITTELS EINES ELEKTRISCHEN SCHWINGKREISES**

CONTROL CIRCUIT FOR A BASE STATION FOR TRANSMITTING ENERGY TO A RECEIVER BY MEANS OF AN ELECTRIC RESONANT CIRCUIT

CIRCUIT DE RÉGULATION D'UNE STATION DE BASE DESTINÉ À LA TRANSMISSION D'UNE ÉNERGIE À UN RÉCEPTEUR À L'AIDE D'UN CIRCUIT LC RÉSONNANT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.12.2014 DE 102014118040**

(43) Veröffentlichungstag der Anmeldung:
**11.10.2017 Patentblatt 2017/41**

(73) Patentinhaber: **Nyxoah SA**
**1435 Mont-St-Guibert (BE)**

(72) Erfinder:
• **MÜLLER, Carsten**
**99097 Erfurt (DE)**
• **KOCH, Timo**
**66839 Schmelz (DE)**

(74) Vertreter: **Patentanwälte Olbricht Buchhold Keulertz**
**Partnerschaft mbB**
**Bettinastraße 53-55**
**60325 Frankfurt am Main (DE)**

(56) Entgegenhaltungen:
WO-A1-2009/051539   WO-A1-2010/059097
WO-A1-2011/008165   WO-A1-2012/148474
WO-A1-2013/164831   WO-A2-2009/091267
US-A1- 2003 040 291   US-A1- 2010 259 109
US-A1- 2012 212 074   US-A1- 2013 200 716
US-A1- 2014 339 910

• **PING SI ET AL: "A Frequency Control Method for Regulating Wireless Power to Implantable Devices", IEEE TRANSACTIONS ON BIOMEDICAL CIRCUITS AND SYSTEMS, IEEE, US, Bd. 2, Nr. 1, 1. März 2008 (2008-03-01), Seiten 22-29, XP011327530, ISSN: 1932-4545, DOI: 10.1109/TBCAS.2008.918284**

**Beschreibung**

**Technisches Gebiet**

Ausführungsbeispiele befassen sich mit einer Regelschaltung für eine Basisstation zum Übertragen einer Energie auf einen Empfänger, vorzugsweise ein medizinisches Implantat, mittels eines elektrischen Schwingkreises und einer Auswerteeinrichtung zum Bestimmen einer von einem elektrischen Schwingkreis einer Basisstation auf einen Empfängerschwingkreis eines medizinischen Implantats übertragenen Energie.

**Hintergrund**

Im Bereich der Medizintechnik kommen in vielen Fällen Implantate zum Einsatz. Bei einem Betrieb solcher Implantate, z. B. zur Stimulation von Nerven oder Muskeln im Körper kann es hilfreich sein, eine Information über einen Stimulationsparameter, welcher an einem Ort der Stimulation auftritt, zu erhalten. Beispielsweise kann es von Bedeutung sein, eine Stärke eines Stimulationsstromes an dem Ort zu kennen, um eine Wirkung des Stromes einschätzen und einstellen zu können. Ferner kann eine Funktionsprüfung des Implantats während oder unmittelbar nach einer Implantation erforderlich sein, was jedoch bisher lediglich unter erschwerten Bedingungen durchführbar ist.

Bei einer Energieübertragung zu Implantaten haben Resonanzverstärker eine weite Verbreitung gefunden, welche einen erleichterten Aufbau und eine vereinfachte Wirkungsweise ermöglichen können. Jedoch kann es in vielen Fällen geschehen, dass ein Schwingkreis des Implantats und ein Schwingkreis einer Primärseite (Basisstation außerhalb des Körpers) in ihrer Position oder Ausrichtung zueinander verändert werden, was eine Energieübertragung ineffizient oder sogar unmöglich machen kann.

In einigen Fällen, beispielsweise bei einem Batteriebetrieb, kann eine Betriebsspannung verhältnismäßig niedrig ausfallen. Da z.B. ein Einsatz eines Schalt-Gleichspannungswandlers ggf. zu Störstrahlung führen kann, kann es sinnvoll sein, die Leistungsendstufen einer Batteriespannung direkt anzupassen, sodass der Gleichspannungswandler womöglich entfallen kann. Ein Gleichspannungswandler kann eine ungünstige Beeinflussung eines Energieverbrauchs, Störstrahlungen oder Probleme bei einer Integration einzelner Funktionseinheiten bei einem lediglich begrenzten Bauvolumen bewirken.

Weiterhin können auch hohe Spannungen aufgrund von Resonanzüberhöhungen (z. B. 2000 V bei einem Reihenschwingkreis) auftreten. Eine Verwendung von Halbleiterbauelementen, die in diesem Spannungsbereich einsetzbar sind, kann dabei oft nicht ohne weiteres möglich sein. Halbleiterbauelemente, die für ein Einschalten der Art hoher Spannungen und Leistungen verwendbar sind, sind in vielen Fällen für einen Bereich der Leistungselektronik bei einer Netzfrequenz von 50 Hz ausgelegt, und können demzufolge für eine Anwendung bei medizinischen Implantaten zu groß ausfallen oder in Bezug auf eine Schaltzeit oder erforderliche Ansteuerleistung problematisch sein.

In Abhängigkeit von einem Frequenzbereich (z. B. 8 MHz) können sich zudem parasitäre Elemente wie Kapazitäten von Bauelementen störend bemerkbar machen, was eine Bauelementauswahl, beispielsweise von Schalttransistoren, erschweren kann. Ferner können sich Schutzschaltungen gegen Überlastungen durch zu hohe Spannungen oder Ströme, die beispielsweise von integrierten Bauelementen umfasst sind, durch eine Aufzehrung von Nutzenergie im Schwingkreis negativ bemerkbar machen.

Gemäß einer konventionellen Lösung kann eine Stimulationswirkung oder eine Funktionstüchtigkeit eines Implantats festgestellt werden, indem die Stimulation anhand einer induzierten Körperreaktion, beispielsweise einer Muskelkontraktion, eingeschätzt wird. Bei Implantaten ohne Energiespeicher kann dabei mit vergleichsweise geringer Stimulationsenergie begonnen, und eine gewünschte Wirkung anhand einer beobachteten Reaktion eines Patienten eingestellt werden. Ferner ist ein Übertragen oder Senden aus dem Implantat heraus mit herkömmlichen Verfahren möglich, jedoch kann es dabei erforderlich sein, dem Implantat die hierfür notwendige Energie mittels eines Energiespeichers zur Verfügung zu stellen.

Gemäß einer weiteren konventionellen Lösung kann eine Steuerung von zu übertragender Energie durch verschiedene Verstärkerkonzepte (Verstärker der Klassen D oder E) erreicht werden. Hierbei können herkömmliche Verfahren, wie z. B. eine Änderung eines Tastverhältnisses bei einer Ansteuerung einer Endstufe, eine Ansteuerung mit Signalen verschiedener Pegel oder eine Steuerung der Betriebsspannung angewendet werden. Auch in anderen Bereichen der Technik, bei denen induktionsbetriebene Schaltungen eingesetzt werden, können derartige Probleme zumindest teilweise relevant sein.

Beispiele einer Übertragung von Energie auf einen Empfänger mittels eines elektrischen Schwingkreises sind aus folgenden Dokumente bekannt: WO 2009/051539 A1, WO 2009/091267 A2, US 2014/339910 A1, US 2012/212074 A1.

Es ist erstrebenswert, ein verbessertes Konzept für eine Leistungsübertragung bei einem System aus Basisstation und medizinischem Implantat und für eine Überwachung von Parametern bei dem medizinischen Implantat zu schaffen.

**Zusammenfassung**

Die Erfindung betrifft eine Regelschaltung für eine Basisstation zum Übertragen einer Energie auf einen Empfänger mittels eines elektrischen Schwingkreises gemäß Anspruch 1. Bevorzugte Ausführungsbeispiele werden durch die abhängigen Ansprüche definiert.

Aspekte oder Ausführungsbeispiele der nachstehenden Beschreibung, die nicht unter den Wortlaut der Ansprüche fallen, sind nicht Teil der Erfindung.

Die Regelschaltung umfasst eine Auswerteeinrichtung, welche dazu ausgebildet ist, um eine auf einen Empfängerschwingkreis des Empfängers übertragene Energie mit einem Energiesollwert zu vergleichen. Die Regelschaltung ist ferner dazu ausgebildet, durch eine auf einem Ergebnis des Vergleichs basierende Veränderung einer Resonanzfrequenz des Schwingkreises einen veränderten Energieeintrag in den Empfängerschwingkreis des Empfängers zu bewirken. Es kann hierdurch unter Umständen eine Einsparung von Energie, und damit ggf. auch ein Auftreten zu hoher Spannungen vermieden werden. Anders ausgedrückt kann hierbei eine Erhöhung einer übertragenen Energie über ein Annähern von Resonanzfrequenzen der Schwingkreise erreicht werden, und hierfür möglicherweise ein Erhöhen einer Eingangsleistung entfallen. Ferner kann möglicherweise ein Bauteilvolumen und Fertigungskosten reduziert werden.

Bei manchen Ausführungsbeispielen ist der Empfänger ein medizinisches Implantat.

Bei einigen Ausführungsbeispielen ist die Regelschaltung dazu ausgebildet, einer Veränderung einer Kopplung des Schwingkreises an den Empfängerschwingkreis durch das Verändern der Resonanzfrequenz des Schwingkreises wenigstens teilweise entgegenzuwirken. Wie erwähnt kann dadurch ein Erhöhen einer Endstufenleistung entfallen, und ein Auftreten zu hoher Spannungen vermieden werden.

Bei manchen Ausführungsbeispielen ist die Regelschaltung dazu ausgebildet, die Resonanzfrequenz des Schwingkreises an eine Eigenfrequenz des Empfängerschwingkreises anzunähern, wenn die übertragene Energie kleiner ist als der Energiesollwert. Dies kann eine erhöhte Effizienz einer Energieübertragung bewirken.

Bei einigen Ausführungsbeispielen ist die Regelschaltung dazu ausgebildet, die Resonanzfrequenz des Schwingkreises von einer Eigenfrequenz des Empfängerschwingkreises zu entfernen, wenn die übertragene Energie größer ist als der Energiesollwert.

Bei manchen Ausführungsbeispielen ist die Regelschaltung dazu ausgebildet, durch ein Verändern eines elektrischen Widerstandes in dem Schwingkreis das Verändern der Resonanzfrequenz zu bewirken.

Bei einigen Ausführungsbeispielen ist die Regelschaltung dazu ausgebildet, durch ein Variieren eines Anregungsstromes das Verändern des elektrischen Widerstandes zu bewirken. Dabei ist der elektrische Widerstand ein effektiver Widerstand des Schwingkreises.

Bei manchen Ausführungsbeispielen ist die Regelschaltung dazu ausgebildet, den elektrischen Widerstand innerhalb eines vorbestimmten Zeitintervalls zu verändern, wobei das Zeitintervall kleiner als eine Schwingungsperiode des Anregungsstromes ist.

Bei einigen Ausführungsbeispielen umfasst die Regelschaltung ferner eine Reihenschaltung eines ersten elektrischen Bauteils und eines zweiten elektrischen Bauteils. Der Schwingkreis ist an eine elektrisch leitende Verbindung zwischen dem ersten elektrischen Bauteil und dem zweiten elektrischen Bauteil gekoppelt, sodass der Anregungsstrom durch ein erstes Eingangssignal des ersten elektrischen Bauteils und/oder ein zweites Eingangssignal des zweiten elektrischen Bauteils bewirkt wird. Dies kann eine aktive Ansteuerung der Regelschaltung ermöglichen.

Bei manchen Ausführungsbeispielen umfasst die Regelschaltung ferner wenigstens eine erste Spannungsquelle. Die erste Spannungsquelle ist dazu ausgebildet, das erste Eingangssignal derart zu erzeugen, dass das erste Eingangssignal wechselweise in dem Zeitintervall einen steigenden oder fallenden Verlauf und in einem weiteren Zeitintervall einen konstanten Verlauf aufweist. Hierdurch kann ein Verändern des effektiven Widerstands des Schwingkreises während des Zeitintervalls bewirkt werden.

Bei einigen Ausführungsbeispielen ist ein Verhältnis des Zeitintervalls zu dem weiteren Zeitintervall kleiner als 1. Hierdurch kann ggf. eine Veränderung des Widerstands derart bewirkt werden, dass ein Einfluss auf eine Schwingkreisgüte dadurch reduziert wird.

Bei manchen Ausführungsbeispielen ist die Regelschaltung dazu ausgebildet, ein Verhältnis des Zeitintervalls zu dem weiteren Zeitintervall zu regeln.

Bei einigen Ausführungsbeispielen ist die Regelschaltung dazu ausgebildet, durch ein Vergrößern des Verhältnisses des Zeitintervalls zu dem weiteren Zeitintervall ein Verringern der Resonanzfrequenz oder durch ein Verringern des Verhältnisses des Zeitintervalls zu dem weiteren Zeitintervall ein Vergrößern der Resonanzfrequenz zu bewirken.

Bei manchen Ausführungsbeispielen umfasst die Regelschaltung ferner wenigstens eine zweite Spannungsquelle. Die zweite Spannungsquelle ist dazu ausgebildet, das zweite Eingangssignal derart zu erzeugen, dass das zweite Eingangssignal wechselweise in dem Zeitintervall einen steigenden oder fallenden Verlauf und in dem weiteren Zeitintervall einen konstanten Verlauf aufweist. Dabei koinzidieren der steigende Verlauf des ersten Eingangssignals mit dem fallenden Verlauf des zweiten Eingangssignals oder der steigende Verlauf des zweiten Eingangssignals mit dem fallenden Verlauf des ersten Eingangssignals.

Bei einigen Ausführungsbeispielen sind das erste elektrische Bauteil ein erster Verstärker, das zweite elektrische Bauteil ein zweiter Verstärker, das erste Eingangssignal eine erste Eingangsspannung und das zweite Eingangssignal eine zweite Eingangsspannung.

Bei manchen Ausführungsbeispielen sind das erste elektrische Bauteil ein erster Transistor, das zweite elektrische Bauteil ein zweiter Transistor, das erste Eingangssignal eine erste Steuerspannung und das zweite Eingangssignal eine zweite Steuerspannung.

Bei einigen Ausführungsbeispielen umfasst die Regelschaltung ferner eine Reihenschaltung eines dritten elektrischen Bauteils und eines vierten elektrischen Bauteils. Dabei koppelt der Schwingkreis an einen Brückenzweig zwischen einer elektrisch leitenden Verbindung zwischen dem ersten elektrischen Bauteil und dem zweiten elektrischen Bauteil und einer elektrisch leitenden Verbindung zwischen dem dritten elektrischen Bauteil und dem vierten elektrischen Bauteil, sodass der Anregungsstrom durch das erste Eingangssignal, das zweite Eingangssignal, ein drittes Eingangssignal des dritten elektrischen Bauteils und/oder ein viertes Eingangssignal des vierten elektrischen Bauteils bewirkt wird.

Erfindungsgemäß umfasst die Regelschaltung ferner ein an den Schwingkreis gekoppeltes resistives Element mit einer zeitlich veränderlichen Resistivität. Das resistive Element umfasst einen Steueranschluss zum Empfangen eines Steuersignals zum Verändern der Resistivität.

Bei einigen Ausführungsbeispielen umfasst die Regelschaltung ferner eine Auswerteeinrichtung. Die Auswerteeinrichtung ist dazu ausgebildet, die übertragene Energie basierend auf einer Modulationseigenschaft eines an den elektrischen Schwingkreis koppelnden Rückkopplungssignals zu ermitteln.

Manche Ausführungsbeispiele richten sich auf eine Basisstation mit einer genannten Regelschaltung. Dabei ist der Schwingkreis dazu ausgebildet, ein Rückkopplungssignal mit einer Modulationseigenschaft zu empfangen, welche Informationen über eine auf den Empfängerschwingkreis des Empfängers übertragene Energie umfasst.

Bei manchen Ausführungsbeispielen ist der Empfänger ein medizinisches Implantat.

Einige Ausführungsbeispiele beziehen sich auf ein System, welches einen Empfänger und eine genannte Basisstation umfasst. Der Empfänger ist dazu ausgebildet, das Energiesignal zu empfangen und das Rückkopplungssignal zu senden.

Gemäß einem weiteren Aspekt beziehen sich Ausführungsbeispiele, die nicht Teil der Erfindung sind, auf eine Auswerteeinrichtung zum Bestimmen einer von einem elektrischen Schwingkreis einer Basisstation auf einen Empfängerschwingkreis eines medizinischen Implantats übertragenen Energie. Die Auswerteeinrichtung umfasst einen Analysator, der dazu ausgebildet ist, eine Modulationseigenschaft eines in dem elektrischen Schwingkreis der Basisstation auftretenden Signals zu bestimmen, und basierend auf der Modulationseigenschaft die auf den Empfängerschwingkreis übertragene Energie zu ermitteln. Hierdurch kann eine Rückkopplungswirkung von dem medizinischen Implantat an die Basisstation ermöglicht werden, bei der eine interne Energieversorgung des Implantats entfallen kann. Ein Zugang zu Informationen über eine Stimulationswirkung oder eine Funktionstüchtigkeit des Implantats kann somit möglicherweise erleichtert werden.

Bei einigen Ausführungsbeispielen umfasst der Analysator ferner einen Demodulator. Der Demodulator ist dazu ausgebildet, die Modulationseigenschaft des Signals durch Demodulieren zu bestimmen.

Bei manchen Ausführungsbeispielen entspricht die Modulationseigenschaft einer Frequenz einer Amplitudenmodulation des Signals.

Bei einigen Ausführungsbeispielen ist der Demodulator dazu ausgebildet, eine Hüllkurve des Signals zu bestimmen. Die Modulationseigenschaft entspricht der Frequenz der Hüllkurve.

Bei manchen Ausführungsbeispielen ist die Auswerteeinrichtung dazu ausgebildet, die Modulationseigenschaft während dem Übertragen der Energie zu bestimmen.

Bei einigen Ausführungsbeispielen entspricht die Modulationseigenschaft einer Frequenzänderung einer Frequenzmodulation des Signals.

Bei manchen Ausführungsbeispielen ist der Demodulator dazu ausgebildet, eine erste Frequenz des Signals an einem ersten Zeitpunkt und eine zweite Frequenz des Signals an einem dem ersten Zeitpunkt folgenden zweiten Zeitpunkt zu messen. Dabei umfasst das Bestimmen der Modulationseigenschaft ein Bestimmen einer Differenz der ersten Frequenz und der zweiten Frequenz.

Bei einigen Ausführungsbeispielen ist die Auswerteeinrichtung dazu ausgebildet, die Modulationseigenschaft unmittelbar nach dem Übertragen der Energie zu bestimmen.

Manche Ausführungsbeispiele beziehen sich auf ein medizinisches Implantat, welches den Empfängerschwingkreis und eine Gleichrichterschaltung umfasst. Die Gleichrichterschaltung ist oder umfasst dabei eine Kapazitätsdiode. Dies kann ein Gleichrichten einer induzierten Spannung ermöglichen.

Einige Ausführungsbeispiele beziehen sich auf eine Basisstation. Die Basisstation umfasst einen elektrischen Schwingkreis zum Übertragen einer Energie auf einen Empfängerschwingkreis eines medizinischen Implantats und eine genannte Auswerteeinrichtung.

Bei manchen Ausführungsbeispielen umfasst die Basisstation ferner einen an den elektrischen Schwingkreis gekoppelten Resonanzverstärker. Der Resonanzverstärker ist dazu ausgebildet, basierend auf einem Steuersignal eine Resonanzfrequenz des elektrischen Schwingkreises zu verändern. Dabei ist der Analysator dazu ausgebildet, das Steuersignal basierend auf der ermittelten Energie zu erzeugen.

Einige Ausführungsbeispiele beziehen sich auf ein System, welches ein medizinisches Implantat und eine genannte Basisstation umfasst.

Gemäß noch einem weiteren Aspekt beziehen sich Ausführungsbeispiele, die nicht Teil der Erfindung sind, auf ein Verfahren zum Bestimmen einer von einem elektrischen Schwingkreis einer Basisstation auf einen Empfängerschwingkreis eines medizinischen Implantats übertragenen Energie. Das Verfahren umfasst ein Bestimmen einer Modulationseigenschaft eines in einen elektrischen Schwingkreis der Basisstation auftretenden Signals. Das Verfahren umfasst zudem ein Ermitteln einer auf den Empfängerschwingkreis übertragene Energie basierend auf der Modulationseigenschaft. Hierdurch kann es ermöglicht werden, ein bei einer Leistungsübertragung auftretendes Echosignal für eine Gewinnung von in dem Implantat auftretenden Parametern zu nutzen. Dabei kann eine interne Energieversorgung des Implantats möglicherweise entfallen.

Gemäß noch einem weiteren Aspekt beziehen sich Ausführungsbeispiele, die nicht Teil der Erfindung sind, auf ein Verfahren zum Übertragen einer Energie auf ein medizinisches Implantat mittels eines elektrischen Schwingkreises. Das Verfahren umfasst ein Vergleichen einer auf einen Empfängerschwingkreis des medizinischen Implantats übertragenen Energie mit einem Energiesollwert. Das Verfahren umfasst zudem ein Bewirken einer Veränderung eines Energieeintrages des Schwingkreises in den Empfängerschwingkreis des medizinischen Implantats durch eine auf einem Ergebnis des Vergleichs basierende Veränderung der Resonanzfrequenz des Schwingkreises . Dies kann eine verbesserte Reaktion auf Störungen bei einer Energieübertragung, wie beispielsweise eine Lageveränderung von Basisstation und Implantat, durch ein gezieltes Regeln der Resonanzfrequenz des Schwingkreises ermöglichen.

Bei manchen Ausführungsbeispielen umfasst das Verfahren optional ein Ermitteln der Energie basierend auf einer Modulationseigenschaft eines an den elektrischen Schwingkreis koppelnden Rückkopplungssignals.

Darüber hinaus schaffen weitere Ausführungsbeispiele, die nicht Teil der Erfindung sind, auch ein Programm oder Computerprogramm mit einem Programmcode zum Durchführen eines der genannten Verfahren, wenn der Programmcode auf einem Computer, einem Prozessor oder einer programmierbaren Hardwarekomponente, wie z.B. einer applikationsspezifischen integrierten Schaltung (ASIC), ausgeführt wird.

Obwohl einige Ausführungsbeispiele am Beispiel eines medizinischen Implantats beschrieben wurden, sind Ausführungsbeispiele nicht auf medizinische Implantate beschränkt, sondern lassen sich auf eine Vielzahl technischer Geräte übertragen, welche dazu ausgebildet sind, Energie zu empfangen.

## Figurenkurzbeschreibung

Exemplarische Ausführungsbeispiele werden nachfolgend bezugnehmend auf die beiliegenden Figuren näher erläutert. Es zeigen:

Fig. 1 ein vereinfachtes Schaltbild eines konventionellen Schwingkreises;

Fig. 2 ein Blockschaltbild einer Regelschaltung für eine Basisstation zum Übertragen einer Energie auf ein medizinisches Implantat mittels eines elektrischen Schwingkreises gemäß einem exemplarischen Ausführungsbeispiel;

Fig. 3 ein Schaltbild eines elektrischen Schwingkreises gemäß einem exemplarischen Ausführungsbeispiel;

Fig. 4 zeitliche Verläufe zweier Eingangssignale, eines effektiven Widerstands und einer Anregungsspannung gemäß einem exemplarischen Ausführungsbeispiel;

Fig. 5 ein Verfahren zum Übertragen einer Energie auf ein medizinisches Implantat mittels eines elektrischen Schwingkreises gemäß einem exemplarischen Ausführungsbeispiel;

Fig. 6 ein Schaltbild eines Schwingkreises eines medizinischen Implantats mit einer Kapazitätsdiode gemäß einem exemplarischen Ausführungsbeispiel;

Fig. 7 einen vereinfachten schematischen Aufbau einer Auswerteeinrichtung gemäß einem exemplarischen Ausführungsbeispiel;

Fig. 8 einen detaillierten schematischen Aufbau einer Auswerteeinrichtung gemäß einem exemplarischen Ausführungsbeispiel;

Fig. 9 zeitliche Verläufe eines Primärsignals, eines Echosignals und einer Modulationseigenschaft des Echosignals gemäß einem exemplarischen Ausführungsbeispiel; und

Fign. 10a,b zeitliche Verläufe eines herkömmlichen Spannungsimpulses und eines Spannungsimpulses gemäß einem exemplarischen Ausführungsbeispiel;

Fig. 11 ein Verfahren zum Bestimmen einer von einem elektrischen Schwingkreis einer Basisstation auf einen Empfängerschwingkreis eines medizinischen Implantats übertragenen Energie gemäß einem exemplarischen Ausführungsbeispiel.

## Beschreibung

Verschiedene exemplarische Ausführungsbeispiele werden nun ausführlicher unter Bezugnahme auf die beiliegenden Zeichnungen beschrieben, in denen einige exemplarische Ausführungsbeispiele dargestellt sind. In den Figuren können die Dickenabmessungen von Linien, Schichten und/oder Regionen um der Deutlichkeit Willen übertrieben dargestellt sein.

Bei der nachfolgenden Beschreibung der beigefügten Figuren, die lediglich einige exemplarische Ausführungsbeispiele zeigen, können gleiche Bezugszeichen gleiche oder vergleichbare Komponenten bezeichnen. Ferner können zusammenfassende Bezugszeichen für Komponenten und Objekte verwendet werden, die mehrfach in einem Ausführungsbeispiel oder in einer Zeichnung auftreten, jedoch hinsichtlich eines oder mehrerer Merkmale gemeinsam beschrieben werden. Komponenten oder Objekte, die mit gleichen oder zusammenfassenden Bezugszeichen beschrieben werden, können hinsichtlich einzelner, mehrerer oder aller Merkmale, beispielsweise ihrer Dimensionierungen, gleich, jedoch gegebenenfalls auch unterschiedlich ausgeführt sein, sofern sich aus der Beschreibung nicht etwas anderes explizit oder implizit ergibt.

Gleiche Bezugszeichen bezeichnen in der gesamten Figurenbeschreibung gleiche oder ähnliche Elemente.

Man beachte, dass ein Element, das als mit einem anderen Element "verbunden" oder "verkoppelt" bezeichnet wird, mit dem anderen Element direkt verbunden oder verkoppelt sein kann oder dass dazwischenliegende Elemente vorhanden sein können. Wenn ein Element dagegen als "direkt verbunden" oder "direkt verkoppelt" mit einem anderen Element bezeichnet wird, sind keine dazwischenliegenden Elemente vorhanden. Andere Begriffe, die verwendet werden, um die Beziehung zwischen Elementen zu beschreiben, sollten auf ähnliche Weise interpretiert werden (z.B., "zwischen" gegenüber "direkt dazwischen", "angrenzend" gegenüber "direkt angrenzend" usw.).

Die Terminologie, die hierin verwendet wird, dient nur der Beschreibung bestimmter Ausführungsbeispiele und soll die Ausführungsbeispiele nicht beschränken. Wie hierin verwendet, sollen die Singularformen "einer," "eine", "eines" und "der, die, das" auch die Pluralformen beinhalten, solange der Kontext nicht eindeutig etwas anderes angibt. Ferner sei klargestellt, dass die Ausdrücke wie z.B. "beinhaltet", "beinhaltend", aufweist" und/oder "aufweisend", wie hierin verwendet, das Vorhandensein von genannten Merkmalen, ganzen Zahlen, Schritten, Arbeitsabläufen, Elementen und/oder Komponenten angeben, aber das Vorhandensein oder die Hinzufügung von einem bzw. einer oder mehreren Merkmalen, ganzen Zahlen, Schritten, Arbeitsabläufen, Elementen, Komponenten und/oder Gruppen davon nicht ausschließen.

Solange nichts anderes definiert ist, haben sämtliche hierin verwendeten Begriffe (einschließlich von technischen und wissenschaftlichen Begriffen) die gleiche Bedeutung, die ihnen ein Durchschnittsfachmann auf dem Gebiet, zu dem die Ausführungsbeispiele gehören, beimisst. Ferner sei klargestellt, dass Ausdrücke, z.B. diejenigen, die in allgemein verwendeten Wörterbüchern definiert sind, so zu interpretieren sind, als hätten sie die Bedeutung, die mit ihrer Bedeutung im Kontext der einschlägigen Technik konsistent ist, solange dies hierin nicht ausdrücklich anders definiert ist.

Ausführungsbeispiele können einen parametrischen Verstärker oder einen Schwingkreis umfassen. Durch Ausführungsbeispiele wird angestrebt, eine Steuerung und Nachführung einer Eigenfrequenz einer Verstärkerendstufe und einer Schwingkreisgüte zu bewirken.

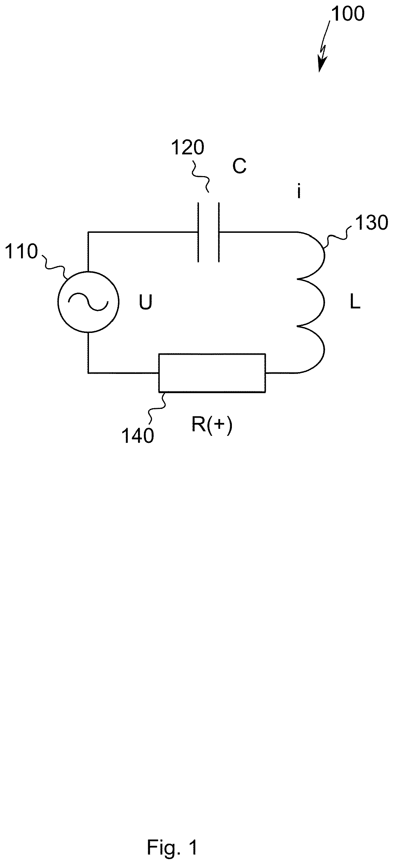

Fig. 1 zeigt einen konventionellen Aufbau eines Schwingkreises 100 gemäß einem herkömmlichen Beispiel. Der Schwingkreis 100 umfasst zunächst eine Spannungsquelle 110, welche eine Wechselspannung bereitstellt. An die Spannungsquelle 110 ist ein Kondensator 120 gekoppelt. An den Kondensator 120 ist eine Spule 130 gekoppelt. Über eine Kopplung der Spule 130 mit der Spannungsquelle 110 wird ein geschlossener Stromkreis gebildet. Ein ohmscher Widerstand 140 kann beispielsweise zu einer summarischen Darstellung von Schwingkreisverlusten dienen. Der Widerstand 140 (R), eine Kapazität C des Kondensators 120 und eine Induktivität L der Spule 130 können dabei zeitlich variabel sein. Bei geschlossenem Stromkreis fällt eine Spannung

$$U = U_C + L \frac{di}{dt} + iR(t)$$

an dem Schwingkreis ab, welche ein Fließen eines Induktionsstromes

$$i = C \frac{dU_C}{dt}$$

bewirkt. Bei zeitlich konstantem Verlustwiderstand R(t)=R kann der Schwingkreis durch die Differenzialgleichung (DGL 1):

$$\frac{1}{L} \frac{dU}{dt} = \frac{1}{LC} i + \frac{R}{L} \frac{\partial i}{\partial t} + \frac{\partial^2 i}{\partial t^2}$$

beschrieben werden. Dabei ergeben sich Terme, welche eine Frequenz des Schwingkreises und eine Dämpfung charakterisieren.

$$\omega_1 = \frac{1}{\sqrt{LC}}$$

Wird der Widerstand R(t), welcher für eine Beschreibung von Gesamtverlusten steht, als zeitabhängig angesetzt, so ergibt sich durch zusätzliche Terme eine erweiterte Differenzialgleichung (DGL 2):

$$\frac{1}{L} \frac{\partial U}{\partial t} = \left( \frac{1}{LC} + \frac{1}{L} \frac{\partial R(t)}{\partial t} \right) + \frac{R(t)}{L} \frac{\partial i}{\partial t} + \frac{\partial^2 i}{\partial t^2}$$

Bei Vergleich der Terme in DGL 1 mit denen in DGL 2 zeigt sich in einem Koeffizienten für eine Kreisfrequenz des Schwingkreises

$$\omega_2 = \sqrt{\frac{1}{LC} + \frac{1}{L} \frac{\partial R(t)}{\partial t}}$$

bei zeitabhängigem Widerstand ein zusätzlicher Term $\partial R(t)/\partial t$. Der Term, welcher die Dämpfung charakterisiert, ist dabei nun ebenfalls zeitabhängig. Hinsichtlich der Dämpfung und einer Schwingkreisgüte Q=ωL/R kann einerseits ein genauer zeitlicher Verlauf der Widerstandsänderung hierbei unbedeutend sein, andererseits kann ein integraler Widerstand über eine Schwingungsperiode auf diese Charakteristiken Einfluss nehmen; beispielsweise kann eine Erhöhung des Integrals des Widerstandes zu einer Verringerung der Schwingkreisgüte Q führen. Nochmals in anderen Worten erklärt, werden die Resonanzfrequenz $\omega_2$ und die Güte Q des Schwingkreises durch die Kapazität C, die Induktivität L und den Widerstand R(t) bestimmt.

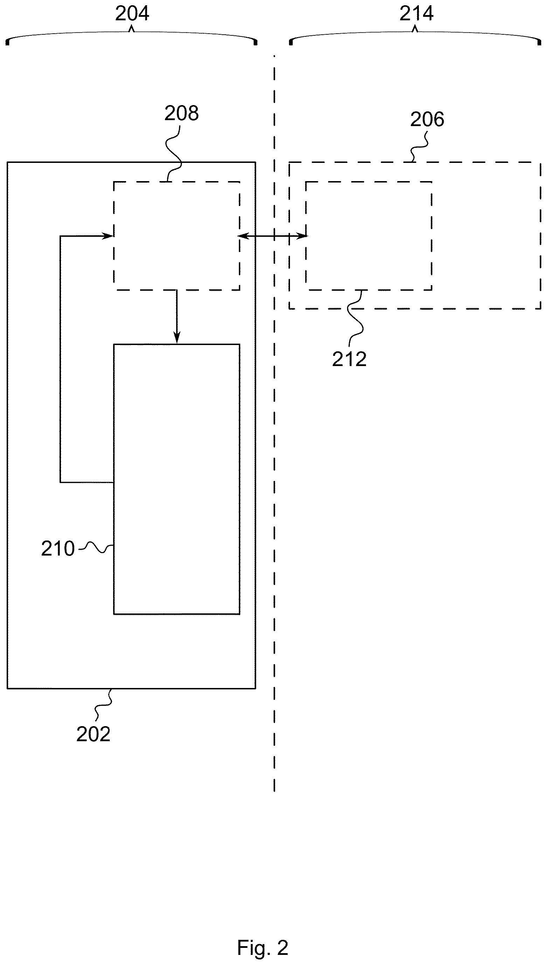

Fig. 2 zeigt eine Regelschaltung 202 für eine Basisstation 204 zum Übertragen einer Energie auf einen Empfänger 206 mittels eines elektrischen Schwingkreises 208 gemäß einem exemplarischen

Ausführungsbeispiel. Lediglich optional vorhandene Elemente sind hierbei durch gestrichelte Linien oder Boxen dargestellt. Die Regelschaltung 202 umfasst eine Auswerteeinrichtung 210, welche dazu ausgebildet ist, um eine auf einen Empfängerschwingkreis 212 des Empfängers 206 übertragene Energie mit einem Energiesollwert zu vergleichen. Die Regelschaltung 202 ist ferner dazu ausgebildet, durch eine auf einem Ergebnis des Vergleichs basierende Veränderung einer Resonanzfrequenz des Schwingkreises 208 einen veränderten Energieeintrag in den Empfängerschwingkreis 212 des medizinischen Implantats 206 zu bewirken. Der Energiesollwert kann sich z.B. aus einem Leistungssollwert ergeben.

Bei manchen Ausführungsbeispielen ist der Empfänger 206 ein medizinisches Implantat 206. Im Folgenden wird der Empfänger 206 als medizinisches Implantat 206 veranschaulicht, was jedoch lediglich exemplarisch zu verstehen ist. Bei anderen Ausführungsbeispielen kann alternativ zu dem medizinischen Implantat 206 eine beliebige andere Art eines Empfängers verwendet werden, der einen Empfängerschwingkreis 212 umfasst.

Das medizinische Implantat 206 ist dabei in einem Körper 214 eines Patienten befindlich. Bei Annäherung der Basisstation 204 an den Körper 214 des Patienten kann eine Wechselwirkung zwischen dem Schwingkreis 208 und den Empfängerschwingkreis 212, oder anders ausgedrückt, ein Leistungsaustausch oder eine induktive Kopplung stattfinden. Der Schwingkreis 208 kann hierbei in die Regelschaltung 202 integriert oder auch auf eine andere Weise mit dieser gekoppelt sein. Somit kann ein Austausch von Signalen oder Informationen zwischen dem Schwingkreis 208 und der Auswerteeinrichtung 210 ermöglicht werden. Bei manchen Ausführungsbeispielen ist die Regelschaltung 202 ferner dazu ausgebildet, einer Veränderung einer Kopplung des Schwingkreises an den Empfängerschwingkreis durch das Verändern der Resonanzfrequenz des Schwingkreises 208 wenigstens teilweise entgegenzuwirken. Eine übertragene Leistung kann dadurch auf eine Art und Weise geregelt werden, welche energieeffizienter sein kann als ein Erhöhen einer Sendeleistung.

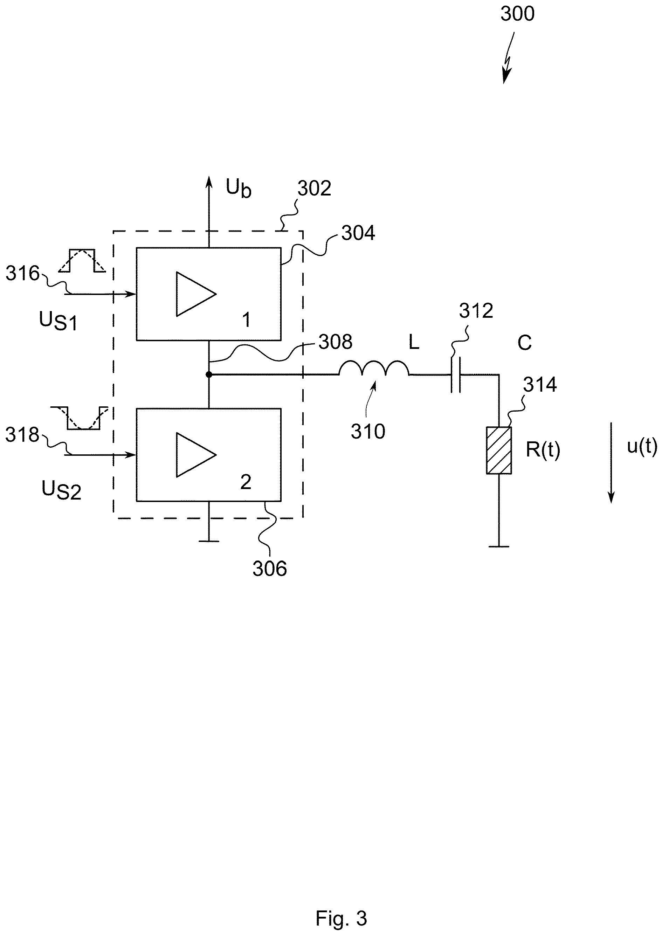

Ein exemplarisches Ausführungsbeispiel eines Schwingkreises 300 ist in Fig. 3 dargestellt. Der Schwingkreis 300 kann beispielsweise mit dem Schwingkreis 208 (vgl. Fig. 2) identisch sein. Ferner kann der Schwingkreis 300, wie Fig. 3 zeigt, als Reihenschwingkreis, bei anderen Ausführungsbeispielen hingegen auch als Parallelschwingkreis ausgeführt sein. Der Schwingkreis 300 ist an eine Halbbrücke 302 oder Reihenschaltung eines ersten elektrischen Bauteils 304 und eines zweiten elektrischen Bauteils 306 gekoppelt, oder von der Halbbrücke 302 umfasst. Genauer gesagt ist das erste elektrische Bauteil 304 zwischen eine Versorgungsspannung $U_b$ und eine elektrisch leitende Verbindung 308, und das zweite elektrische Bauteil 306 zwischen die elektrisch leitende Verbindung 308 und Masse gekoppelt. Zwischen die Verbindung 308 und Masse ist eine Reihenschaltung eines induktiven Elements 310 (z.B. einer Spule) und eines kapazitiven Elements 312 (z.B. eines Kondensators) geschaltet. Ein zeitabhängiger Widerstand 314 R(t) repräsentiert dabei auftretende Energieverluste in dem Schwingkreis 300. Dem ersten elektrischen Bauteil 304 wird ein erstes Eingangssignal 316, und dem zweiten elektrischen Bauteil 306 ein zweites Eingangssignal 318 zugeführt. Das erste Eingangssignal 316 und das zweite Eingangssignal 318 können von einer Ansteuervorrichtung bereitgestellt werden. Bei dem hier gezeigten Ausführungsbeispiel sind das erste elektrische Bauteil 304 ein erster Verstärker, das zweite elektrische Bauteil 306 ein zweiter Verstärker, das erste Eingangssignal 316 eine erste Eingangsspannung $U_{s1}$, und das zweite Eingangssignal 318 eine zweite Eingangsspannung $U_{s2}$. Verschiedene Klassen von Verstärkern können hierbei verwendet werden. Bei einem Verstärker der Klasse E kann jedoch ein Koppelfaktor einer Sender- und einer Empfängerspule erheblichen Einfluss auf Betriebseigenschaften des Verstärkers haben. Dies kann bei der Anwendung in medizinischen Implantaten, bei der es häufig zu Lageänderungen der Spulen zueinander kommen kann, lediglich unter erhöhtem Aufwand beherrschbar sein. Dies kann jedoch bedeuten, dass ein bei diesem Verstärkertyp erhöhter Wirkungsgrad wieder aufgehoben wird. Bei manchen Ausführungsbeispielen kommen daher auch Verstärker der Klasse D zum Einsatz. Bei einem anderen Ausführungsbeispiel können das erste elektrische Bauteil 304 ein erster Transistor (z. B. Feldeffekttransistor), das zweite elektrische Bauteil 306 ein zweiter Transistor, das erste Eingangssignal 316 eine erste Gate- oder Steuerspannung, und das zweite Eingangssignal 318 eine zweite Gate- oder Steuerspannung sein. Durch Verändern der Gate- oder Steuerspannungen können die Leitfähigkeiten der elektrischen Bauteile 304; 306 beeinflusst werden, wodurch sich an der elektrisch leitenden Verbindung 308 eine zeitabhängige Anregungsspannung ergibt. Die Anregungsspannung kann einen Strom i(t) bewirken, welcher eine zeitlich variable Anregung des Schwingkreises 300 bewirken kann.

Gemäß einigen Ausführungsbeispielen wird durch ein Verändern des Widerstandes 314 in dem Schwingkreis 300 das Verändern der Resonanz- oder Eigenfrequenz des Schwingkreises 300 bewirkt. Der Widerstand 314 kann ein effektiver Widerstand des Schwingkreises 300 sein. Es kann beispielsweise durch ein Variieren eines Anregungsstromes das Verändern des elektrischen Widerstandes 314 bewirkt werden. Der Anregungsstrom wiederum kann durch die erste Eingangsspannung 316 ($U_{s1}$) und die zweite Eingangsspannung 318 ($U_{s2}$) bewirkt werden. Somit wird es in einigen Ausführungsbeispielen ermöglicht, die Resonanzfrequenz des Schwingkreises 300 an eine Eigenfrequenz des Empfängerschwingkreises anzunähern, wenn die übertragene Energie kleiner ist als der Energiesollwert, oder von der Eigenfrequenz des Empfängerschwingkreises zu entfernen, wenn die übertragene Energie größer ist als der Energiesollwert.

Es kann dabei möglich sein, dass die zeitliche Ableitung $\partial R(t)/\partial t$ des Widerstandes 314 die Frequenz, und ein zeitlicher Mittelwert des Widerstandes 314 die Güte Q=ωL/R des Schwingkreises (vgl. hierzu auch Fig. 1) beeinflusst. Somit kann mit einer periodischen Änderung des Widerstandes 314 in dem Schwingkreis 300 auch eine Änderung des Gütefaktors Q des Schwingkreises 300 verbunden sein, welche wiederum mit einer Änderung einer erhaltenen Amplitude der von dem Schwingkreis 300 auf den Empfängerschwingkreis übertragenen Energie einhergehen kann. Das hierin beschriebene Prinzip kann auch zu einer Steuerung der übertragenen Energie dienen. Folglich kann es erstrebenswert sein, die Güteänderung unter einen vorbestimmten Grenzwert zu reduzieren und in die Steuerung der zu übertragenden Energie einzubeziehen. Bei manchen Ausführungsbeispielen kann dies erreicht werden, indem der Schwingkreis 300 auf eine Frequenz unterhalb der gewünschten Resonanzfrequenz des Empfängerschwingkreises abgestimmt wird. Die Güte Q kann ein Maß für ein Schwingungsverhalten oder Schwingkreisverluste eines angeregten Schwingkreises sein. Dabei kann eine Erhöhung der Güte Q einer Verringerung einer Schwingungsdämpfung entsprechen. Tritt im zeitlichen Mittel eine Erhöhung des Verlustwiderstandes 314 gleichzeitig zu einer Erhöhung der Resonanzfrequenz des Schwingkreises 300 auf, so kann eine Erhöhung eines induktiven Widerstandes $X_L = \omega L$ in der Beziehung Q = ωL/R der Erhöhung des Widerstandes 314 entgegenwirken. Anders ausgedrückt können die Änderungen in eine gemeinsame Richtung erfolgen, wodurch sich die Güte Q geringfügiger ändert. Bei der Änderung der Resonanzfrequenz des Schwingkreises 300 kann, mit anderen Worten, eine Vergrößerung eines Betrages der Widerstandsänderung $\partial R(t)/\partial t$ einen erheblichen Einfluss haben, und ein definierter zeitlicher Verlauf der Widerstandsänderung vernachlässigbar sein. Hierdurch kann eine Mehrzahl von Ausgestaltungen ermöglicht werden, wovon eine im Folgenden näher erläutert wird.

Bei einem Ausführungsbeispiel kann die bereits beschriebene Schaltungsanordnung für Verstärker, bzw. Verstärkerendstufen (Halbbrückenschaltung 302) oder eine Vollbrückenschaltung, ferner auch eine Drossel (Choke-Spule) eingesetzt werden. Eine periodische Änderung des Verlustwiderstandes 314 R(t) kann durch mehrere Varianten ermöglicht werden. Bei einer ersten Variante werden der bereits vorhandene erste Verstärker 304 und zweite Verstärker 306 verwendet. Bei einer zweiten Variante kann zusätzlich oder alternativ ein Schaltelement an den Schwingkreis gekoppelt werden. Dies kann beispielsweise ein resistives Element mit einer zeitlich veränderlichen Resistivität sein, welche dem Widerstand 314 entspricht. Dabei fällt über dem resistiven Element eine durch einen Pfeil gekennzeichnete Verlustspannung u(t) ab, welche die Anregungsspannung beeinflusst. Das resistive Element kann einen Steueranschluss zum Empfangen eines Steuersignals zum Verändern der Resistivität umfassen.

Bei der ersten Variante, bei der die Halbbrückenschaltung 302 verwendet wird, kann eine Vereinfachung des Aufbaus möglich sein. Die Ansteuerung des ersten Verstärkers 304 und des zweiten Verstärkers 306 kann beispielsweise mit einer Ansteuerung für einen regulären Verstärkermodus kombiniert werden. Um die gewünschte Widerstandsänderung $\partial R(t)/\partial t$ zu erreichen, kann z.B. eine Steilheit der Schaltflanken der Eingangsspannungen $U_{s1}$, $U_{s2}$ zur Ansteuerung der Endstufe variiert, oder Phasenverschiebungen der Eingangsspannungen vorgenommen werden. Ein Verändern der Eingangsspannungen bewirkt eine zeitlich veränderliche Leitfähigkeit der Verstärker 304; 306, und somit eine zeitabhängige, in Fig. 3 über dem Schwingkreis 300 abfallende Anregungsspannung u(t), welche wiederum einen Anregungsstrom hervorruft. Durch diesen Anregungsstrom kann der Widerstand 314 R(t) verändert werden. Bei der zweiten Variante können ggf. über das zusätzliche oder alternative Schaltelement (z.B. Metalloxid-Halbleiter-Feldeffekttransistor, MOSFET) positive Spannungen lediglich in einem vordefinierten Bereich auftreten, für welchen optional vorhandene, interne Schutzschaltungen ausgelegt sind.

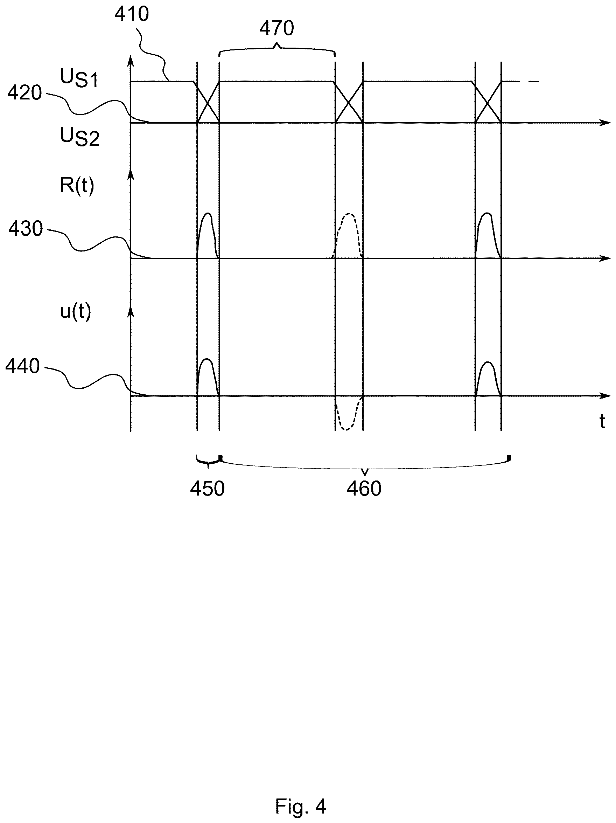

Fig. 4 stellt die bereits erläuterten Zusammenhänge anhand von verschiedenen Signalverläufen dar. Hierbei sind die zeitlichen Verläufe der ersten Eingangsspannung $U_{s1}$ (410), der zweiten Eingangsspannung $U_{s2}$ (420), des Widerstandes R(t) (430) und der Anregungsspannung u(t) (440) gezeigt. Bei einem Ausführungsbeispiel ist dabei die Regelschaltung dazu ausgebildet, den Widerstand R(t) innerhalb eines vorbestimmten Zeitintervalls 450 zu verändern. Dabei ist das vorbestimmten Zeitintervalls 450 kleiner als eine Schwingungsperiode des Anregungsstromes. Die Schwingungsperiode des Anregungsstromes kann dabei einer Schwingungsperiode 460 der Anregungsspannung entsprechen. Die Regelschaltung kann ferner eine Spannungsquelle zum Erzeugen der ersten oder zweiten Eingangsspannung aufweisen. Die Spannungsquelle kann dabei dazu ausgebildet sein, die erste oder zweite Eingangsspannung derart zu erzeugen, dass diese wechselweise in dem Zeitintervall 450 einen steigenden oder fallenden Verlauf,

und in einem weiteren Zeitintervall 470 einen konstanten Verlauf aufweist. Ein Verhältnis des Zeitintervalls 450 zu dem weiteren Zeitintervall 470 kann dabei kleiner als 1 sein, was auch Fig. 4 verdeutlicht. Ferner kann die Regelschaltung dazu ausgebildet sein, das Verhältnis des Zeitintervalls 450 zu dem weiteren Zeitintervall 470 zu regeln. Es kann somit bei einigen Ausführungsbeispielen ermöglicht werden, durch ein Vergrößern des Verhältnisses des Zeitintervalls 450 zu dem weiteren Zeitintervall 470 ein Verringern der Resonanzfrequenz oder durch ein Verringern des Verhältnisses des Zeitintervalls 450 zu dem weiteren Zeitintervall 470 ein Vergrößern der Resonanzfrequenz zu bewirken. Anders ausgedrückt bewirkt das Vergrößern des Verhältnisses ein Verringern der Widerstandsänderung $\partial R(t)/\partial t$, und gemäß dem bereits genannten Zusammenhang

$$\omega_2 = \sqrt{\frac{1}{LC} + \frac{1}{L}\frac{\partial R(t)}{\partial t}}$$

ein Verringern der Resonanzfrequenz $\omega_2$, und umgekehrt.

Bei einigen Ausführungsbeispielen können dabei konstante zeitliche Verläufe der Eingangsspannungen $U_{s1}$, $U_{s2}$, zeitlich koinzidieren. Zudem kann ein steigende Verlauf der ersten Eingangsspannung $U_{s1}$ mit einem fallenden Verlauf der zweiten Eingangsspannung $U_{s2}$ und umgekehrt zeitlich koinzidieren. Mit anderen Worten werden der erste Verstärker und der zweite Verstärker gegenphasig mit den jeweiligen Eingangsspannungen angesteuert. Dabei können die Schaltflanken im Anstieg veränderlich sein, was durch temporäres Verlangsamen einer Umschaltdauer (dies entspricht einem bewussten "Verschlechtern" der Schalteigenschaften) der Verstärkerstufe erreicht werden kann. Daraus kann sich ein vorbestimmter variabler zeitlicher Verlauf des Verlustwiderstandes R(t) (in Fig. 4 dargestellt als durchgezogene und gestrichelte Kurven) während des Umschaltvorganges zwischen beiden Verstärkern ergeben. Der Widerstandverlauf kann seinerseits einen Spannungsverlauf u(t) an den Verstärkerelementen mit positiver und negativer Polarität verursachen. Eine ähnliche Wirkung kann gemäß der zweiten Variante mit dem zusätzlichen diskreten Schaltelement mit einem Verhalten eines zeitlich variablen Widerstandes erreicht werden. Die Schaltflanken der Eingangsspannungen können dabei unverändert bleiben. Je nach Phasenlage kann es dabei möglich sein, lediglich positive Spannungsüberhöhungen (durchgezogene Kurve) zu erhalten. Dabei kann eine einfachere Kontrolle der Spannungsüberhöhungen auch unter Verwendung von konventionellen Bauelementen erreicht werden. Anders ausgedrückt können vergleichsweise erhöhte Spannungen lediglich in einer Polarität auftreten, was bewirken kann, dass beispielsweise von MOSFETs umfasste Bodydioden nur wenig oder gar keine Nutzenergie aufzehren.

Bei einem Ausführungsbeispiel kann optional zu der Halbbrückenschaltung eine Drosselspule verwendet werden. Ferner kann bei einem anderen Ausführungsbeispiel die Regelschaltung eine Reihenschaltung eines dritten elektrischen Bauteils und eines vierten elektrischen Bauteils umfassen. Der Schwingkreis ist dabei an einen Brückenzweig zwischen einer elektrisch leitenden Verbindung zwischen dem ersten elektrischen Bauteil und dem zweiten elektrischen Bauteil und einer elektrisch leitenden Verbindung zwischen dem dritten elektrischen Bauteil und dem vierten elektrischen Bauteil gekoppelt, so dass der Anregungsstrom durch das erste Eingangssignal, das zweite Eingangssignal, ein drittes Eingangssignal des dritten elektrischen Bauteils und/oder ein viertes Eingangssignal des vierten elektrischen Bauteils bewirkt wird. Anders ausgedrückt kann alternativ zu der Halbbrückenschaltung eine Vollbrückenschaltung verwendet werden. Die Halbbrückenschaltung oder die Vollbrückenschaltung können jeweils mit Komplementärstufen oder einem High-Side-Treiber ausgeführt sein. Somit kann möglicherweise eine z.B. 4-fach erhöhte Ausgangsleistung bezogen auf jeweils gleiche Versorgungsspannung erreicht werden. Das Schaltelement kann ferner in Kombination mit der Halbbrücke oder der Vollbrücke eingesetzt werden.

Nochmals mit anderen Worten erklärt kann die Regelschaltung dazu ausgebildet sein Parameter eines Schwingkreises in Kombination mit einer elektronischen Ansteuerung und einer entsprechenden Software (Mikroprozessor/Computer) zu steuern. Dabei kann ein Ausgleich einer frequenzabhängigen Verstärker-Ausgangsamplitude infolge verschiedener Eigen- oder Resonanzfrequenzen des Schwingkreises erfolgen. Ausführungsbeispiele können unter Umständen einen Betrieb von Resonanzverstärkern mit verringerter, unstabilisierter Versorgungsspannung, beispielsweise ausgehend von einer Batterie, ermöglichen. Eine Ausgangsleistung oder übertragene Energie kann somit durch eine Steuerung von Blindelementen (Reaktanzen) gesteuert werden, was eine Reduzierung von Verlusten bewirken kann. Ein zeitlicher Verlauf einer Änderung der Güte Q (Verlustwiderstand) kann hierbei beliebig sein. Bei manchen Ausführungsbeispielen kann eine Anpassung einer Verstärkercharakteristik an vergleichsweise kleine, unstabilisierte Versorgungsspannungen erfolgen, was möglicherweise eine verbesserte Nutzung von Batterie-Versorgungsspannungen erlauben kann. Parameter können beispielsweise durch Softwaresteuerung eingestellt werden. Hierbei kann eine Adaption an verschiedene Anforderungen vorgenommen werden, wobei Hardwareänderungen ggf. entfallen können. Unerwünschte Wirkungen von Schutzschaltungen (z.B. der Bodydiode eines MOSFETs) können dabei eventuell verringert werden.

Die genannten Ausführungsbeispiele können für verschiedene Zwecke eingesetzt werden, beispielsweise für eine Frequenznachführung von Schwingkreisen, bei der eine Umschaltung von Reaktanzen möglicherweise entfallen kann. Auch kann eine Leistungsanpassung oder eine Steuerung einer Eigenresonanz von Verstärkerstufen vorgenommen werden. Manche Ausführungsbeispiele können auch im Zusammenhang mit durchstimmbaren Hochfrequenz-Verstärkern implementiert werden.

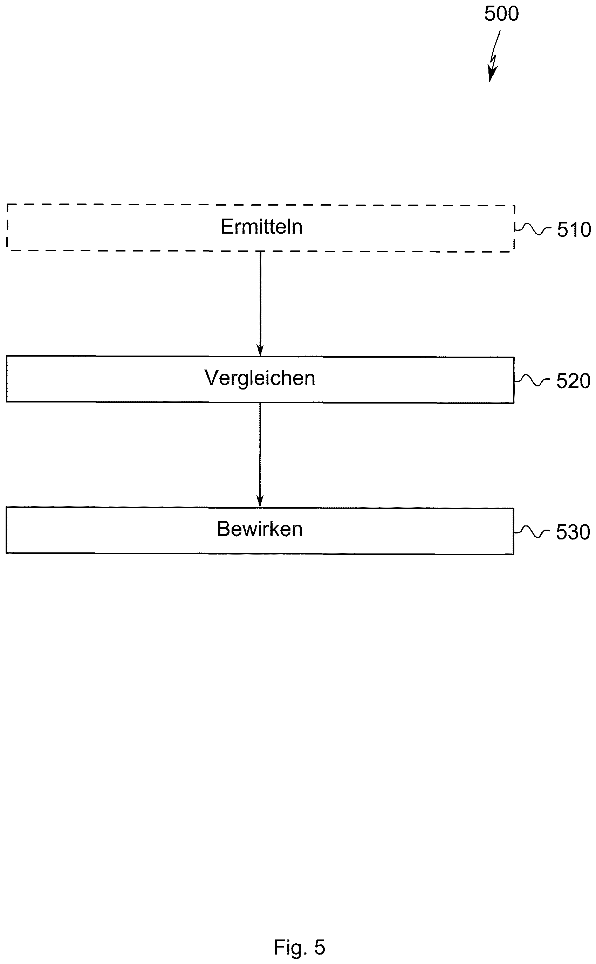

Fig. 5 zeigt ein Verfahren 500 zum Übertragen einer Energie auf ein medizinisches Implantat mittels eines elektrischen Schwingkreises, gemäß einem exemplarischen Ausführungsbeispiel. Das Verfahren 500 umfasst ein Vergleichen 520 einer auf einen Empfängerschwingkreis des medizinischen Implantats übertragenen Energie mit einem Energiesollwert. Das Verfahren 500 umfasst zudem ein Bewirken 530 einer Veränderung eines Energieeintrages in den Empfängerschwingkreis des medizinischen Implantats durch eine auf einem Ergebnis des Vergleichs basierende Veränderung der Resonanzfrequenz des Schwingkreises. Dies kann eine verbesserte Reaktion auf Störungen bei einer Energieübertragung, wie beispielsweise eine Lageveränderung von Basisstation und Implantat, durch ein gezieltes Regeln der Resonanzfrequenz des Schwingkreises ermöglichen.

Bei manchen Ausführungsbeispielen umfasst das Verfahren 500 optional ein Ermitteln 510 der Energie basierend auf einer Modulationseigenschaft eines an den elektrischen Schwingkreis koppelnden Rückkopplungssignals. Das Ermitteln 510 kann dabei dem Vergleichen 520 vorausgehen.

Nochmals mit anderen Worten erklärt, können Ausführungsbeispiele eine Steuerung der übertragenen Energiemenge während des Betriebes ermöglichen. Im Vergleich zu herkömmlichen Methoden können dabei dynamische Verluste unter Umständen verringert werden. Zudem kann bei Ausführungsbeispielen eine Anpassung der zu übertragenden magnetischen Energie erfolgen. Hierbei kann ggf. lediglich eine Versorgungsspannungsquelle mit unstabilisierter Versorgungsspannung verwendet werden (Batteriebetrieb), und dadurch Verluste möglicherweise verringert werden. Ausführungsbeispiele können zudem eine Steuerung der Eigenfrequenz von Schwingkreisen erlauben. Anforderungen an Bauelemente, eine Anzahl an Bauelementen, erforderlicher Bauraum oder auch ein Einfluss parasitärer Kapazitäten von Schaltelementen lassen sich hierdurch möglicherweise reduzieren. Durch eine parametrische Steuerung gemäß Ausführungsbeispielen kann unter Umständen eine Reduzierung der Spannungsbelastung für Halbleiterbauelemente infolge von Resonanzüberhöhungen erfolgen. Außerdem kann es bei einer Einstellung verschiedener Resonanzfrequenz möglich werden, dass zusätzliche Bauelemente entfallen können.

Weiterhin kann die Regelschaltung bei manchen Ausführungsbeispielen ferner eine Auswerteeinrichtung umfassen. Die Auswerteeinrichtung ist dazu ausgebildet, die übertragene Energie basierend auf einer Modulationseigenschaft eines an den elektrischen Schwingkreis koppelnden Rückkopplungssignals zu ermitteln. Optional kann die Regelschaltung auch von einer Basisstation umfasst sein. Ferner kann der Schwingkreis dazu ausgebildet sein, das Rückkopplungssignal mit der Modulationseigenschaft zu empfangen. Die Modulationseigenschaft kann dabei Informationen über eine auf den Empfängerschwingkreis des medizinischen Implantats übertragene Energie umfassen. Noch weitere Ausführungsbeispiele beziehen sich ferner auf ein System, welches die genannte Basisstation und das medizinische Implantat umfasst. Das medizinische Implantat ist dazu ausgebildet, das Energiesignal zu empfangen und das Rückkopplungssignal zu senden. Die Begriffe "empfangen" und "senden" können dabei im weiteren Sinne neben dem herkömmlichen Verständnis auch weitere Arten von Kopplung umfassen, bei der eine Datenübertragung stattfinden kann. Beispielsweise können der Schwingkreis der Basisstation und der Empfängerschwingkreis des medizinischen Implantats auch durch modulierte Rückstreuung (Lastmodulation) aneinander gekoppelt sein.

Gemäß konventionellen Lösungen wird eine Funktionsweise passiver Implantate, z.B. für eine Nervenstimulation, anhand applizierter Reaktionen eines Patienten überprüft. Dieser Kontrollmechanismus kann jedoch subjektiv und darüber hinaus ungenau sein, da lediglich Ja-Nein-Aussagen getroffen werden können. Mit anderen Worten kann der Patient auf die Stimulation reagieren oder nicht. Degenerative oder alterungsbedingte Veränderungen an der Elektronik des Implantats können auf diese Weise nur erschwert festgestellt werden. Eine Erhebung genauerer Daten von Parametern, welche in dem Implantat vorliegen, beispielsweise Resonanzfrequenz oder Stimulationsintensität, oder körpereigener Parameter des Patienten oder Probanden, beispielsweise Gewebeimpedanz (diese kann einer Abschlussimpedanz des Implantats entsprechen) kann hierbei wünschenswert sein. Eine konventionelle Lösung ist eine Verwendung eines aktiven Implantats. Hierbei kann jedoch zusätzlicher Bauraum für eine Energiequelle erforderlich sein.

Zum Ermitteln der auf das medizinische Implantat übertragenen Energie werden im Rahmen von Ausführungsbeispielen mehrere Varianten vorgeschlagen. Gemäß einer ersten Variante kann eine Restenergie in dem Implantat genutzt werden, welche unmittelbar nach einem Stimulationsimpuls oder einer anderen gezielten äußeren Anregung vorhanden ist. Die Restenergie kann nach einem vordefinierten Muster abklingen. Dabei können elektromagnetische Schwingungen mit einer charakteristischen Frequenz abgestrahlt werden. Gemäß einer zweiten Variante kann eine Energie genutzt werden, welche während der Stimulation dem Implantat zugeführt wird. Hierbei kann eine vorbestimmte Frequenz genutzt werden, welche während der Stimulation abgestrahlt wird.

Bei einigen Ausführungsbeispielen umfasst das medizinische Implantat eine Diode. Die Diode kann eine von einer anliegenden Sperrspannung abhängige Sperrschichtkapazität aufweisen. Die nach einem Ende des Stimulationsimpulses im Implantat abklingende Energie (auch als "Rückflanke" bezeichnet) oder die während des Stimulationsimpulses anliegende Energie erzeugt eine bestimmte, z.B. mit einer gegebenen Zeitkonstanten abklingende Sperrspannung an der Diode. Dies kann zu einer Frequenzmodulation der abgestrahlten Hochfrequenz führen. Während der Abstrahlung kann sich diese Frequenz ändern, sodass eine Information darüber, welcher Strom i(t) in einem Zeitraum der Rückflanke oder in einem Verlauf des Stimulationsimpulses geflossen ist oder fließt.

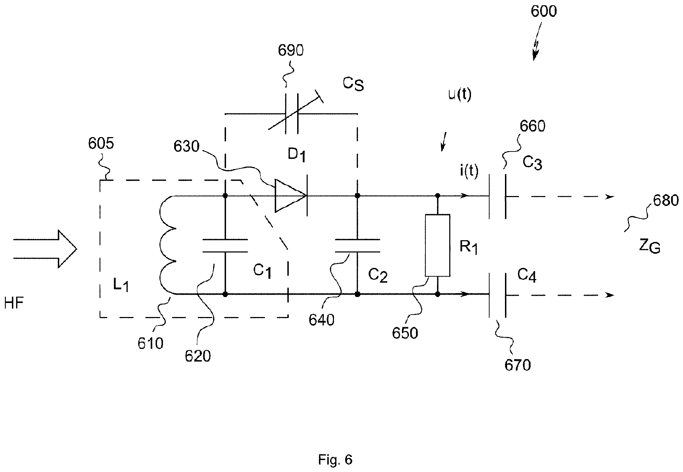

Fig. 6 zeigt ein Schaltbild für ein mögliches exemplarisches Ausführungsbeispiel eines medizinischen Implantats 600, welches zu einer elektrischen Stimulation von Muskeln oder Nerven dienen kann. Eine Parallelschaltung eines induktiven Elements 610 mit einem ersten kapazitiven Element 620 bildet einen Parallelschwingkreis 605. Dabei weist das induktive Element 610 eine Induktivität Li, und das erste kapazitive Element 620 eine Kapazität $C_1$ auf. Über eine Diode 630 ist ein Kopplungspunkt des ersten kapazitiven Elements 620 mit einem Kopplungspunkt eines zweiten kapazitiven Elements 640, und ein dem Kopplungspunkt abgewandter weiterer Kopplungspunkt des ersten kapazitiven Elements 620 mit einem weiteren Kopplungspunkt des zweiten kapazitiven Elements 640 verbunden. Dabei weist das zweite kapazitive Element 640 eine Kapazität $C_2$ auf. Eine Sperrrichtung der Diode 630 weist dabei von dem zweiten kapazitiven Element 640 zu dem ersten kapazitiven Element 620. Bei einigen Ausführungsbeispielen ist die Diode 630 eine Gleichrichterdiode, oder anders ausgedrückt, von einer Gleichrichterschaltung umfasst, und kann eine Gleichrichtung einer von außerhalb des Körpers empfangenen Hochfrequenzspannung (z.B. 8 MHz) bewirken, aus welcher ein Stimulationsimpuls gebildet werden kann.. Die Diode 630 kann dabei eine Kapazitätsdiode sein. In Fig. 6 ist dies durch eine der Diode 630 parallel geschaltete, zeitlich variable Kapazität 690 (Cs) verdeutlicht. Je nach Wahl der Diode 630 kann dabei ein physisches Bauelement für die zeitlich variable Kapazität 690 entfallen.

Ein Kopplungspunkt des zweiten kapazitiven Elements 640 ist mit einem Kopplungspunkt eines resistiven Elements 650, und ein dem Kopplungspunkt abgewandter weiterer Kopplungspunkt des zweiten kapazitiven Elements 640 mit einem weiteren Kopplungspunkt des resistiven Elements 650 verbunden. Das resistive Element 650 weist dabei eine Resistivität $R_1$ auf. Ein Kopplungspunkt des resistiven Elements 650 ist über ein drittes kapazitives Element 660, und ein dem Kopplungspunkt abgewandter weiterer Kopplungspunkt des resistiven Elements 650 über ein viertes kapazitives Element 670 an ein Gewebe 680 (z.B. Muskeln oder Nerven) kontaktiert. Eine Gewebeimpedanz beträgt dabei $Z_G$. Das dritte kapazitive Element 660 weist eine Kapazität $C_3$, und das vierte kapazitive Element 670 eine Kapazität $C_4$ auf.

Der Parallelschwingkreis 605 empfängt eine, z.B. von einem Schwingkreis einer Basisstation übertragene, Energie mit einer vorgegebenen Frequenz (HF) und Amplitude, welche beispielsweise von einem Resonanzverstärker erzeugt werden kann. Die Energie kann, abhängig von Parametervorgaben, für die Stimulation moduliert sein, z.B. mit der Zeitdauer der Stimulation. Für eine Gewinnung des Stimulationsimpulses selbst erfolgt eine Demodulation oder Gleichrichtung unter Verwendung der Diode 630. Über einen durch das zweite kapazitive Element 640 und das resistive Element 650 gebildeten Dämpfungsabschnitt erfolgt eine Glättung, und über das jeweils als Koppelkondensator verwendete dritte und vierte kapazitive Element 660; 670 eine potenzialfreie Auskopplung einer induzierten Spannung u(t) in das Gewebe 680. Die zeitabhängige induzierte Spannung u(t) bewirkt dabei einen zeitabhängigen Strom i(t). Je nach Stärke der induzierten Spannung variiert der Strom, und durch die Kapazitätsdiode 630 abhängig von dem Strom ein Schwingungsverhalten des Parallelschwingkreises 605, oder anders ausgedrückt, die Eigenfrequenz des Parallelschwingkreises 605. Ein hierbei rückgestrahltes Signal kann dabei Informationen umfassen, welche Rückschlüsse auf beispielsweise eine Implantat-Eigenfrequenz oder eine Impedanz des Gewebes 680 an einem Ort des Implantats 600 erlauben kann.

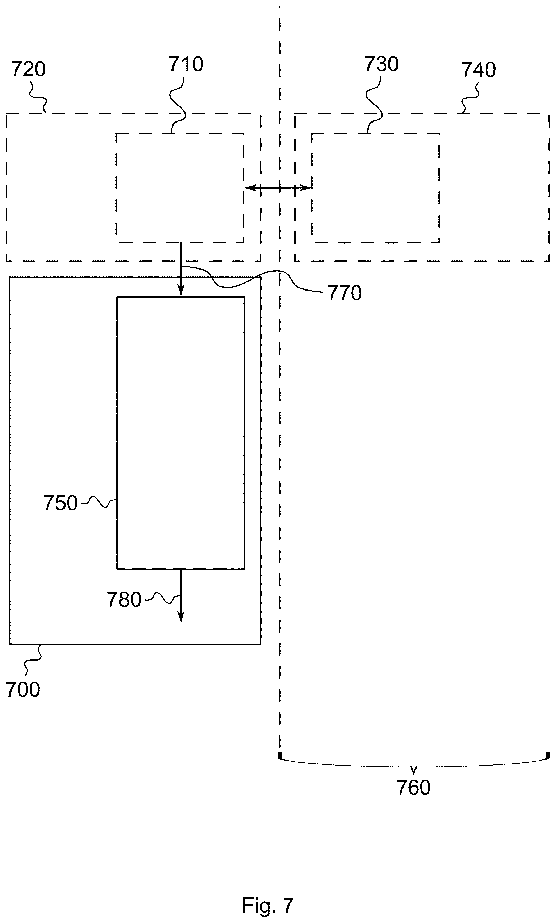

Fig. 7 zeigt ein exemplarisches Ausführungsbeispiel für eine Auswerteeinrichtung 700 zum Bestimmen einer von einem elektrischen Schwingkreis 710 einer Basisstation 720 auf einen Empfängerschwingkreis 730 eines medizinischen Implantats 740 übertragenen Energie. Wiederum sind optional vorhandene Komponenten durch gestrichelte Linien und gestrichelte Boxen dargestellt. Der Empfängerschwingkreis 730 kann dabei mit dem Parallelschwingkreis 605, und das medizinische Implantat 740 mit dem medizinischen Implantat 600 (vgl. Fig. 6) identisch sein. Die Auswerteeinrichtung 700 umfasst einen Analysator 750, welcher dazu ausgebildet ist, eine Modulationseigenschaft eines in dem elektrischen Schwingkreis 710 der Basisstation 720 auftretenden Signals zu bestimmen, und basierend auf der Modulationseigenschaft die auf den Empfängerschwingkreis 730 übertragene Energie zu ermitteln. Das medizinische Implantat 740 kann sich dabei im Inneren eines menschlichen oder tierischen Körpers 760 befinden. Der Schwingkreis 710 und der Empfängerschwingkreis 730 sind drahtlos, beispielsweise induktiv, aneinander gekoppelt. Somit kann durch den Schwingkreis 710 ein Übertragen einer Energie auf den Empfängerschwingkreis 730 erfolgen. Umgekehrt erfolgt über die induktive Kopplung auch eine Rückwirkung vom Implantat auf den Empfängerschwingkreis. Ferner ist der Schwingkreis 710 an den Analysator 750 gekoppelt, sodass eine Übertragung 770 von Information, beispielsweise des Signals, von dem Schwingkreis 710 an die Auswerteeinrichtung 750 erfolgen kann. Der Analysator 750 kann auch dazu ausgebildet sein, eine bzgl. der Auswerteeinrichtung 700 interne oder externe Ausgabe 780 der ermittelten Energie vorzunehmen. Die Basisstation 720 kann, wie in Fig. 7 gezeigt, extern mit der Auswerteeinrichtung 700 koppelbar sein, oder alternativ die Auswerteeinrichtung 700 und den Schwingkreis 710 umfassen.

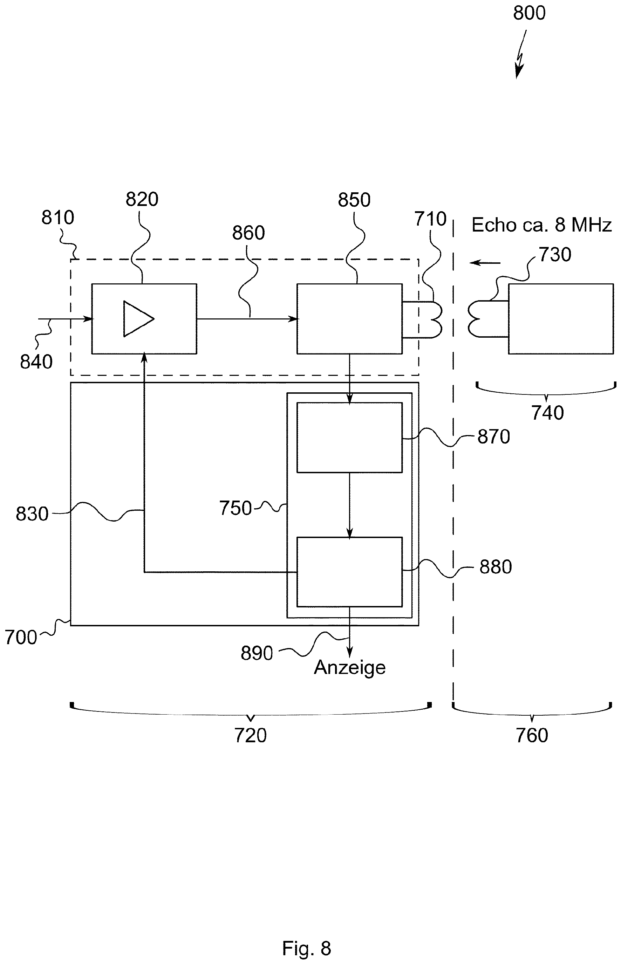

Ein weiteres Blockdiagramm einer Auswerteeinrichtung 700 gemäß einem noch detaillierteren exemplarischen Ausführungsbeispiel ist in Fig. 8 gezeigt. Komponenten, welche eine Entsprechung in Fig. 7 aufweisen, sind mit gleichen Bezugszeichen gekennzeichnet, und hierin nicht nochmals erklärt. Es wird vielmehr lediglich auf die Unterschiede eingegangen. Die Basisstation 720 umfasst in Fig. 8 die Auswerteeinrichtung 700 und eine Regelstrecke 810, welche mit dem Schwingkreis 710 gekoppelt ist. Die Regelstrecke 810 umfasst einen Resonanzverstärker 820, welcher dazu ausgebildet ist, ein Steuersignal 830 von der Auswerteeinrichtung 700, oder genauer gesagt, dem Analysator 750, und eine Führungsgröße 840, beispielsweise eine Eingangsspannung, zu empfangen. Der Resonanzverstärker 820 ist ferner dazu ausgebildet, basierend auf dem Steuersignal 830 eine Resonanzfrequenz des Schwingkreises 710 zu verändern. Die Regelstrecke umfasst ferner einen Entkopplungsabschnitt 850, welcher eine Stellgröße 860, z.B. eine Anregungsspannung oder Anregungsstrom von dem Resonanzverstärker 820 empfängt und eine entkoppelte Größe an den Schwingkreis 710 überträgt. Über den Entkopplungsabschnitt kann zudem das in dem Schwingkreis 710 auftretende Signal an den Analysator 750 übertragen werden. Weiterhin kann der Schwingkreis 710 von einer Endstufe des Resonanzverstärkers 820 umfasst sein. Das im Schwingkreis 710 auftretende Signal kann beispielsweise durch den Empfängerschwingkreis 730 hervorgerufen werden, und ein Echosignal mit einer Frequenz von mehreren Megahertz, z.B. 8 MHz sein. Bei einigen Ausführungsbeispielen können das medizinische Implantat 740 und die Basisstation 720 von einem gemeinsamen System 800 umfasst sein.

Der Analysator 750 umfasst ferner einen Demodulator 870, der dazu ausgebildet ist, die Modulationseigenschaft des Signals durch Demodulieren zu bestimmen. Ferner umfasst der Analysator 750 einen Mikroprozessor 880, welcher dazu ausgebildet ist, eine Information über die Modulationseigenschaft von dem Demodulator 870 zu empfangen. Der Mikroprozessor 880 ist ferner dazu ausgebildet, basierend auf der Modulationseigenschaft die auf den Empfängerschwingkreis 730 übertragene Energie zu ermitteln. Zusätzlich kann der Mikroprozessor 880 dazu ausgebildet sein, ein Anzeigesignal 890 mit Informationen über die Energie an eine Anzeigeeinrichtung bereitzustellen. Auch kann der Mikroprozessor 880 oder der Analysator 750 dazu ausgebildet sein, basierend auf der ermittelten Energie das Steuersignal 830 zu erzeugen und an den Resonanzverstärker 820 bereitzustellen.

Anders ausgedrückt kann die Basisstation 720 Module zur Energieversorgung und Ansteuerung des Implantats 740 umfassen. Der Resonanzverstärker 820 führt dabei die Energieversorgung und Ansteuerung des Schwingkreises 710 (oder, mit anderen Worten, einer Primärspule des Schwingkreises 710) durch. Der Schwingkreis 710 kann für den Verstärker und auch für eine Erfassung des Echosignals genutzt werden. Hierzu kann mittels des Entkopplungsabschnitts 850 eine Entkopplung dieser beiden Funktionen vorgenommen werden, wodurch eine bidirektionale Übertragung ermöglicht werden kann. Der Entkopplungsabschnitt 850 (beispielsweise in Form einer Weiche ausgeführt) kann auch eine hinreichende Unterdrückung eines Ausschwingens des Schwingkreises 710 nach der Rückflanke des Anregungs- oder Stimulationsimpulses bewirken.

Das Echosignal kann in einem Ausführungsbeispiel demoduliert, und ein daraus folgendes niederfrequenteres Signal an den Mikroprozessor 880 bereitgestellt werden. Der Mikroprozessor 880 kann zudem eine Analog-Digital-Wandlung durchführen. Eine Auswertung des Echosignals kann z.B. über eine herkömmliche Anzeigeeinheit erfolgen oder für eine Regelung verwendet werden. Die Regelung kann beispielsweise wünschenswert sein, wenn ein Aufrechterhalten einer definierten Stimulation unter wechselnden örtlichen Bedingungen (z.B. Schwankung der Kopplung zwischen Schwingkreis 710 und Empfängerschwingkreis 730 infolge von Bewegungen oder sich ändernden Gewebeimpedanzen) angestrebt wird. Weiterhin kann es möglich sein, dieses Konzept derart individuell anzupassen, dass sich mehrere Anwendungsmöglichkeiten ergeben, wie z.B. eine einfache Funktionsprüfung des Implantats 740 oder auch komplexere Mess- und Überwachungsfunktionen.

Alternativ zu einer Verwendung eines Demodulators zur Demodulation eines frequenzmodulierten Signals kann es auch bei manchen Ausführungsbeispielen möglich sein, die von dem Implantat rückgestrahlte Frequenz direkt zu bestimmen. Hierzu kann beispielsweise ein Mikroprozessor mit erhöhter Geschwindigkeit verwendet werden, der dazu ausgebildet ist, nach einem Prinzip eines Zählfrequenzmessers zu arbeiten.

Nochmals mit anderen Worten erklärt kann ein passives Implantat zur Nerven- oder Muskelstimulation einen Empfangsschwingkreis und eine Komponente zur Gleichrichtung und Glättung einer Spannung umfassen, um aus einer äußerlichen, hochfrequenten Anregung durch die Basisstation einen Stimulationsimpuls zu generieren. Des Weiteren kann ein spannungsbegrenzendes Bauelement, z.B. eine Zenerdiode, verwendet werden, wodurch eine Überstimulation vermieden, und somit ein verbesserter Schutz eines Probanden möglich sein kann. Alternativ können Gleichrichtung und Spannungsbegrenzung durch eine Kapazitätsdiode erfolgen. Die Kapazität der Diode kann sich über eine an ihr anliegende Spannung ändern. Eine erreichbare Diodenspannung kann mittels einer Stärke der Ansteuerung durch die Basisstation und durch die jeweilige Abschlussimpedanz (Gewebeimpedanz) des passiven Implantats vorgegeben sein. Die sich ausbildende Kapazität kann somit von Stimulationsstärke und Gewebeimpedanz abhängen. Eine Kapazitätsänderung im Empfängerschwingkreis kann jedoch auch eine Veränderung der Abstimmung zwischen Basisstation und Implantat hervorrufen. Bei einer Fehlabstimmung durch die sich verändernde Kapazität des Empfängerschwingkreises kann möglicherweise weniger Energie zum Implantat gelangen, was eine Spannungs- und somit Kapazitätsänderung in eine entgegengesetzte Richtung zur Folge haben kann. Je nach Wahl der Kapazitätsdiode kann diese Wechselwirkung zwischen Implantat und Basisstation in einem Frequenzband erfolgen, welches sich von der Stimulationsfrequenz beispielsweise um wenigstens eine oder auch mehrere Größenordnungen (Faktor 10 oder höher) unterscheiden kann. Es kann so rückwirkend eine Modulation der Ansteuerung im Schwingkreis der Basisstation erfolgen. Nach entsprechender Demodulation kann über die, beispielsweise vermittels eines Mikroprozessors, gemessene Frequenz eine Modulationseigenschaft des Echosignals in der Basisstation verfügbar gemacht werden. Überdies kann es möglich sein, durch Verwendung einer Kapazitätsdiode mit einer bestimmten Durchbruchspannung die Spannung im Implantat automatisch zu limitieren, und damit einen verbesserten Schutz des Probanden zu ermöglichen.

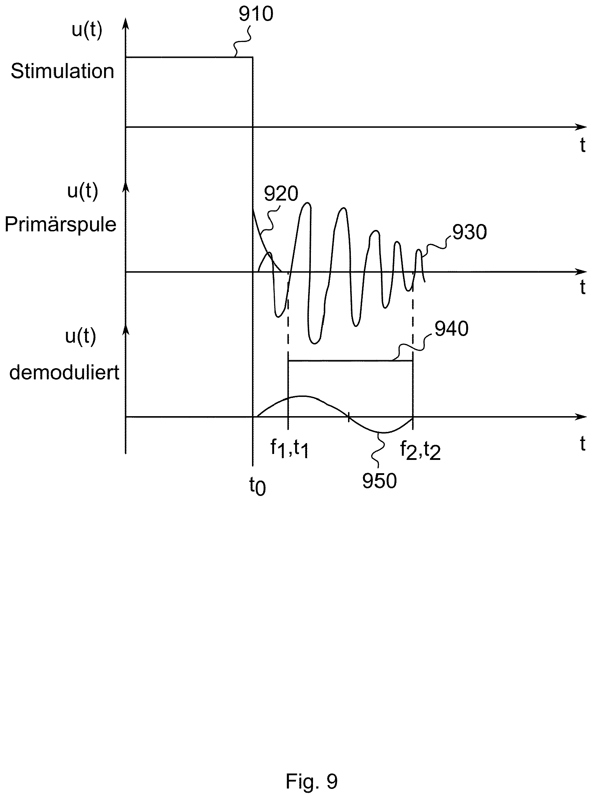

Wie oben beschrieben, kann zum Ermitteln der auf das medizinische Implantat übertragenen Energie gemäß der zuvor genannten ersten Variante eine Restenergie in dem Implantat genutzt werden, welche unmittelbar nach einem Stimulationsimpuls oder einer anderen gezielten äußeren Anregung vorhanden ist. Fig. 9 zeigt hierbei auftretende zeitliche Verläufe von Signalen, hier in Form von Spannungen dargestellt, an verschiedenen Komponenten der Basisstation. Es erfolgt zunächst eine äußere Anregung 910 (Stimulation) in Form eines über eine vorbestimmte Zeit konstanten Spannungswertes (Rechteckspannung), welche zu einem Zeitpunkt $t_0$ endet. In der Primärspule des Schwingkreises tritt ab dem Zeitpunkt $t_0$ ein abklingendes Primärsignal 920 sowie das Echosignal 930 auf. Bei manchen Ausführungsbeispielen entspricht die Modulationseigenschaft einer Frequenzänderung einer Frequenzmodulation des Echosignals 930. Zur Bestimmung der Frequenzänderung kann der Demodulator dazu ausgebildet sein, eine erste Frequenz $f_1$ des Echosignals 930 an einem ersten Zeitpunkt $t_1$ und eine zweite Frequenz $f_2$ an einem zweiten Zeitpunkt $t_2$ zu messen. Der zweite Zeitpunkt $t_2$ folgt dabei dem ersten Zeitpunkt $t_1$ zeitlich. Die beiden Zeitpunkte können ein Messfenster 940 eingrenzen. Das Bestimmen der Modulationseigenschaft umfasst ein Bestimmen einer Differenz der ersten Frequenz $f_1$ und der zweiten Frequenz $f_2$. Wie aus Fig. 9 ersichtlich wird, kann bei manchen Ausführungsbeispielen die Auswerteeinrichtung dazu ausgebildet sein, die Modulationseigenschaft nach dem Übertragen der Energie (oder, mit anderen Worten, nach einem Überschreiten des Zeitpunkts $t_0$) zu bestimmen. Das Messfenster 940 kann somit zeitlich auf oder unmittelbar auf den Zeitpunkt $t_0$ folgen. Das Echosignal 930 weist eine sich zeitlich ändernde Frequenz auf, sodass durch den Demodulator hieraus ein demoduliertes Signal 950 ermittelbar ist. Die übertragene Energie hängt mit anderen Worten von einer Größe der Differenz der ersten Frequenz $f_1$ und der zweiten Frequenz $f_2$ oder einem Verlauf des demodulierten Signals 950 ab.

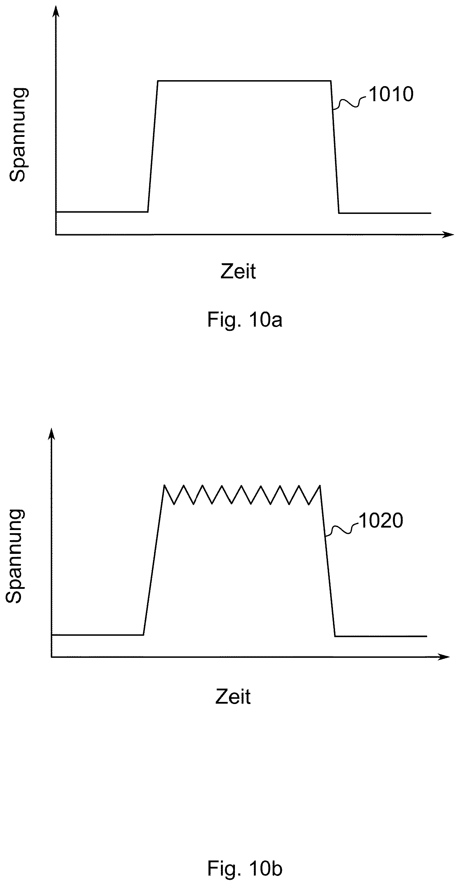

Gemäß der zuvor genannten zweiten Variante kann eine Energie genutzt werden, welche während der Stimulation dem Implantat zugeführt wird. Mit anderen Worten kann dabei das Messfenster 940 zeitlich mit der äußeren Anregung 910 wenigstens teilweise koinzidieren. Fig. 10a zeigt einen zeitlichen Verlauf eines herkömmlichen, näherungsweise rechteckförmigen (abschnittsweise konstanten), gleichgerichteten Stimulationsimpulses 1010. Im Vergleich hierzu zeigt Fig. 10b einen zeitlichen Verlauf eines Stimulationsimpulses 1020, welcher bei Anwendung der zweiten Variante in dem Schwingkreis auftreten kann. Mit anderen Worten handelt es sich bei dem Stimulationsimpuls 1020 um ein amplitudenmoduliertes Signal. Nochmals anders ausgedrückt kann der Stimulationsimpuls 1020 eine Überlagerung zweier Signale darstellen, deren Frequenzen sich um wenigstens eine Größenordnung unterscheiden.

Bei manchen Ausführungsbeispielen kann die Modulationseigenschaft einer Frequenz einer Amplitudenmodulation des Signals entsprechen. Der Demodulator kann ferner dazu ausgebildet sein, eine Hüllkurve des Signals zu bestimmen. Die Modulationseigenschaft kann dabei einer Frequenz der Hüllkurve entsprechen. Die Auswerteeinrichtung kann bei einigen Ausführungsbeispielen dazu ausgebildet sein die Modulationseigenschaft während dem Übertragen der Energie zu bestimmen. Hierdurch kann es möglich sein, auch solche Frequenzen direkt zu generieren, welche eine unmittelbare Auswertung mit einem Mikroprozessor erlauben.

Durch die Verwendung der zweiten Variante können während einer Stimulation zeitgleich die eingestellten Stimulationsparameter mit denen an dem Implantat auftretenden Stimulationsparametern (z.B. Stimulationsstrom) verglichen und ggf. nachgeregelt werden. Somit kann unter Umständen auch eine schnellere Nachregelung der Stimulationsparameter bei Bewegung des Patienten oder einer Verkippung des Empfängerschwingkreises des Implantats zu dem Schwingkreis der Basisstation (oder deren jeweiligen Spulen zueinander) ermöglicht werden. Hierbei kann es möglich sein, einen Bauteilbedarf bei dem Implantat zu reduzieren, da durch die Kapazitätsdiode mehrere Funktionen zeitgleich übernommen werden können, welche ansonsten mehrere separate Bauteile (z.B. Diode für die Gleichrichtung und eine zusätzliche Zenerdiode) erfordern könnten. Die Kapazitätsdiode kann als gleichrichtendes Bauelement verwendet werden, und gleichzeitig die Spannung limitieren. Durch eine verringerte Durchbruchspannung kann dabei eventuell ein höherer Schutz des Patienten ermöglicht werden. Eine Auswertung des Signals bei der zweiten Variante kann ferner durch einen vereinfachten Einsatz von elektronischen Bauteilen erfolgen. Es erfolgt bei der Basisstation einer Amplitudenmodulation sowie eine Demodulation. Die Anregungsfrequenz kann dabei beispielsweise um das 30-fache über einer Frequenz des demodulierten Signals liegen. Dabei kann die Frequenz des demodulierten Signals im Bereich von einigen 100 kHz liegen. Ein Messen dieser Frequenz kann bereits mit vereinfachten und Energiesparenderen Mikrocontrollern im Rahmen einer erforderlichen Genauigkeit möglich sein.

Die Anwendung der ersten und zweiten Variante kann mittels des in Fig. 8 erläuterten Ausführungsbeispiels erfolgen. Mit anderen Worten kann dieses Ausführungsbeispiel eine Regelschleife umfassen. Über das Steuersignal 830 können die Stimulationsparameter eingestellt, und ferner deren Einhaltung mithilfe der Modulationseigenschaft überwacht werden. Dabei kann ein Schutz des Patienten erfolgen, da durch die entstehende Frequenz des Echosignals bei zu hoher Stimulationsenergie ein Eingriff zur Reduzierung der Energie auch über die Basisstation 720 möglich werden kann. Hierdurch kann die Anzahl erforderlicher, implantatseitiger Bauteile möglicherweise reduziert werden.

Für die Demodulation kann ferner ein so genannter Laufzeit-Demodulator, beispielsweise für einen Frequenzbereich von 6-10 MHz, verwendet werden. Dieser kann durch digitale Bauelemente, z.B. ein NAND-Gatter gebildet werden.

Bei manchen Ausführungsbeispielen ändern sich Eigenschaften des Gewebes und sonstige Umgebungsbedingungen des Implantats lediglich auf vergleichsweise großen Zeitskalen. Dies kann zur Folge haben, dass sich bei einem vorbestimmten Zyklus die Stimulationsparameter anhand der antizipierten Umgebungsbedingungen für die jeweils nächste Stimulation mit erhöhter Genauigkeit einstellen lassen. Ein Zyklus kann dabei zunächst eine Stimulation (initiale Anregung), weiterhin eine Echoauswertung und Parameterkorrektur, und wiederum eine Stimulation, Echoauswertung und Parameterkorrektur, usw. umfassen. Eine Messung kann dabei mehrere Schritte aufweisen. Zunächst erfolgt eine initiale Anregung des Implantats mit vergleichsweise geringer Energie (Echo-Empfangsmodus). Anhand des Echosignals wird eine grundlegende Funktionsprüfung des Implantats durchgeführt. Steuergrößen für die Energiezuführung zum Implantat werden unter Beachtung der gewünschten Stimulationsparameter mittels eines Mikroprozessors eingestellt. Es folgt eine Stimulation, und beispielsweise bei Ende des Stimulationsimpulses ein erneuter Empfang des Echosignals. Anhand des Echosignals kann eine Überprüfung oder Korrektur der Stimulationsparameter vorgenommen werden. Somit kann wiederum eine Einstellung der Steuergrößen erfolgen.

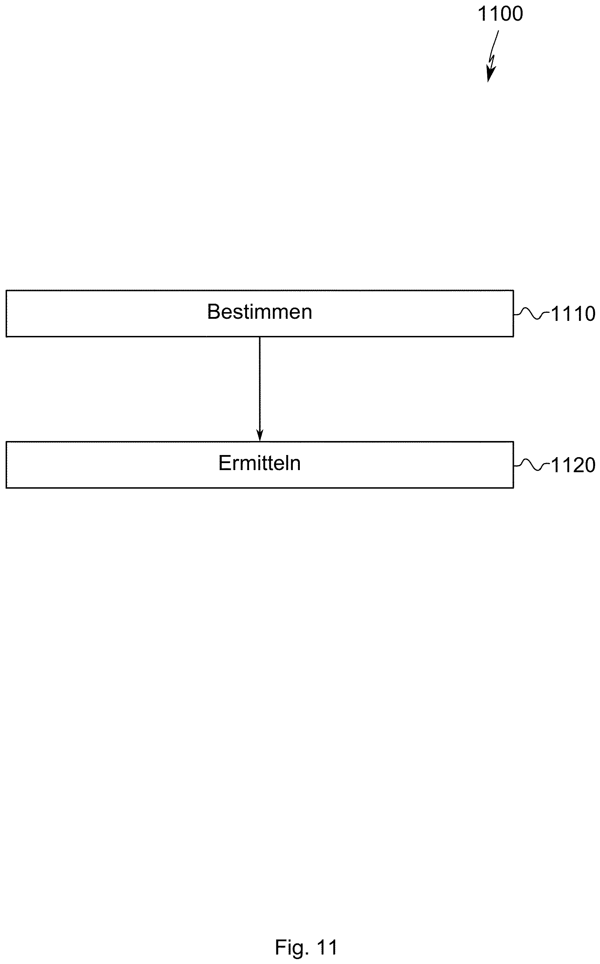

Fig. 11 zeigt ein exemplarisches Ausführungsbeispiel für ein Verfahren 1100 zum Bestimmen einer von einem elektrischen Schwingkreis einer Basisstation auf einen Empfängerschwingkreis eines medizinischen Implantats übertragenen Energie. Das Verfahren 1100 umfasst ein Bestimmen 1110 einer Modulationseigenschaft eines in einen elektrischen Schwingkreis der Basisstation auftretenden Signals. Das Verfahren 1100 umfasst zudem ein Ermitteln 1120 einer auf den Empfängerschwingkreis übertragene Energie basierend auf der Modulationseigenschaft. Hierdurch kann es ermöglicht werden, ein bei einer Energieübertragung auftretendes Echosignal für eine Gewinnung von in dem Implantat auftretenden Parametern zu nutzen. Dabei kann eine interne Energieversorgung des Implantats möglicherweise entfallen.

Manche Ausführungsbeispiele beziehen sich ferner auf ein aktives Implantat, bei dem ein Energiespeicher entfallen kann. Das Implantat und die Basisstation können von einem gemeinsamen System umfasst sein. Hierbei können Regelschleifen (Closed-Loop) zur Beeinflussung von biologischen oder technischen Parametern in biologischen oder technischen Systemen implementiert sein. Manche Ausführungsbeispiele können eventuell zu einer erhöhten Belastbarkeit des Systems beitragen. Es kann ferner eine kontinuierliche oder quasi-kontinuierliche Messung von Parametern und ein passives Auslesen derselben erfolgen. Ferner kann ein vergleichsweise hoher Grad an Software für einen Betrieb von Closed-Loop-Systemen verwendet werden, was eine Miniaturisierung verbessern kann. Durch eine elektrische Messmethode können ggf. Eine Messung in dem Implantat und eine exaktere Einstellbarkeit von Stimulationsparameter erfolgen. Ein zusätzlicher Energiebedarf zu einer Gewinnung des Echosignals kann dabei reduziert werden oder sogar entfallen. Ferner kann ein Bedarf an elektronischen Bauelementen möglicherweise reduziert werden. Bei manchen Ausführungsbeispielen können Messsignale (demodulierte Signale) durch die Basisstation ausgewertet werden. Dies kann eine automatische Nachführung von Stimulationsparametern im Körper des Probanden ermöglichen, eine Elektronik des Implantats vereinfachen, oder auch eine Intelligenz externer Elektronik verbessern. Eine Auswertung des Messsignals könnte zudem vereinfacht werden. Auch könnte eine Funktionsprüfung von Implantaten während und nach einer Implantation möglich werden, wobei eine unbeabsichtigte Stimulation möglicherweise vermieden werden kann. Ausführungsbeispiele können beispielsweise zu einer Funktionsprüfung von Implantaten, eine Einstellung und Kontrolle definierter Stimulationsparameter im Körper oder einer Bestimmung einer Gewebeimpedanz (Muskelgewebe, Fett) im Körper verwendet werden.

Die in der vorstehenden Beschreibung, den nachfolgenden Ansprüchen und den beigefügten Figuren offenbarten Merkmale können sowohl einzeln wie auch in beliebiger Kombination für die Verwirklichung eines Ausführungsbeispiels in ihren verschiedenen Ausgestaltungen von Bedeutung sein und implementiert werden.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar.

Je nach bestimmten Implementierungsanforderungen können Ausführungsbeispiele in Hardware oder in Software implementiert sein. Die Implementierung kann unter Verwendung eines digitalen Speichermediums, beispielsweise einer Floppy-Disk, einer DVD, einer Blu-Ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einer programmierbaren Hardwarekomponente derart zusammenwirken können oder zusammenwirken, dass das jeweilige Verfahren durchgeführt wird.

Eine programmierbare Hardwarekomponente kann durch einen Prozessor, einen Computerprozessor (CPU = Central Processing Unit), einen Grafikprozessor (GPU = Graphics Processing Unit), einen Computer, ein Computersystem, einen anwendungsspezifischen integrierten Schaltkreis (ASIC = Application-Specific Integrated Circuit), einen integrierten Schaltkreis (IC = Integrated Circuit), ein Ein-Chip-System (SOC = System on Chip), ein programmierbares Logikelement oder ein feldprogrammierbares Gatterarray mit einem Mikroprozessor (FPGA = Field Programmable Gate Array) gebildet sein.

Das digitale Speichermedium kann daher maschinen- oder computerlesbar sein. Manche Ausführungsbeispiele umfassen also einen Datenträger, der elektronisch lesbare Steuersignale aufweist, die in der Lage sind, mit einem programmierbaren Computersystem oder einer programmierbare Hardwarekomponente derart zusammenzuwirken, dass eines der hierin beschriebenen Verfahren durchgeführt wird. Ein Ausführungsbeispiel ist somit ein Datenträger (oder ein digitales Speichermedium oder ein computerlesbares Medium), auf dem das Programm zum Durchführen eines der hierin beschriebenen Verfahren aufgezeichnet ist.

Allgemein können Ausführungsbeispiele als Programm, Firmware, Computerprogramm oder Computerprogrammprodukt mit einem Programmcode oder als Daten implementiert sein, wobei der Programmcode oder die Daten dahin gehend wirksam ist bzw. sind, eines der Verfahren durchzuführen, wenn das Programm auf einem Prozessor oder einer programmierbaren Hardwarekomponente abläuft. Der Programmcode oder die Daten kann bzw. können beispielsweise auch auf einem maschinenlesbaren Träger oder Datenträger gespeichert sein. Der Programmcode oder die Daten können unter anderem als Quellcode, Maschinencode oder Bytecode sowie als anderer Zwischencode vorliegen.

Die oben beschriebenen exemplarischen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche beschränkt sei.

## Patentansprüche

1. Regelschaltung (202) für eine Basisstation (204) zum Übertragen einer Energie auf einen Empfänger (206) mittels eines elektrischen Schwingkreises (208; 300), umfassend:

   einen elektrischen Schwingkreis (208; 300);
   eine Auswerteeinrichtung (210), welche dazu ausgebildet ist, um eine auf einen Empfängerschwingkreis (212) des Empfängers (206) übertragene Energie mit einem Energiesollwert zu vergleichen,
   wobei die Regelschaltung (202) dazu ausgebildet ist, durch eine auf einem Ergebnis des Vergleichs basierende Veränderung einer Resonanzfrequenz des Schwingkreises (208; 300) einen veränderten Energieeintrag in den Empfängerschwingkreis (212) des Empfängers (206) zu bewirken,
   wobei die Auswerteeinrichtung (210) dazu ausgebildet ist, die übertragene Energie basierend auf einer Modulationseigenschaft eines an den elektrischen Schwingkreis (208; 300) koppelnden Rückkopplungssignals zu ermitteln,
   wobei die Regelschaltung (202) dazu ausgebildet ist, einer Veränderung einer Kopplung des Schwingkreises (208; 300) an den Empfängerschwingkreis (212) durch das Verändern der Resonanzfrequenz des Schwingkreises (208; 300) wenigstens teilweise entgegenzuwirken,
   wobei die Regelschaltung (202) dazu ausgebildet ist, die Resonanzfrequenz des Schwingkreises (208; 300) von einer Eigenfrequenz des Empfängerschwingkreises (212) zu entfernen, wenn die übertragene Energie größer ist als der Energiesollwert,
   **dadurch gekennzeichnet, dass** die Regelschaltung ein an den Schwingkreis (208; 300) gekoppeltes resistives Element mit einer zeitlich veränderlichen Resistivität (314) umfasst, welches einen Steueranschluss zum Empfangen eines Steuersignals zum Verändern der Resistivität (314) umfasst.

2. Regelschaltung (202) gemäß Anspruch 1, welche dazu ausgebildet ist, die Resonanzfrequenz des Schwingkreises (208; 300) an eine Eigenfrequenz des Empfängerschwingkreises (212) anzunähern, wenn die übertragene Energie kleiner ist als der Energiesollwert.

3. Regelschaltung (202) gemäß einem der Ansprüche 1 bis 3, welche dazu ausgebildet ist, durch ein Verändern eines elektrischen Widerstandes (314) in dem Schwingkreis (208; 300) das Verändern der Resonanzfrequenz zu bewirken.

4. Regelschaltung (202) gemäß Anspruch 3, welche dazu ausgebildet ist, durch ein Variieren eines Anregungsstromes das Verändern des elektrischen Widerstandes (314) zu bewirken, wobei der elektrische Widerstand (314) ein effektiver Widerstand des Schwingkreises (208; 300) ist.

5. Regelschaltung (202) gemäß Anspruch 4, welche dazu ausgebildet ist, den elektrischen Widerstand (314) innerhalb eines vorbestimmten Zeitintervalls (450) zu verändern, wobei das Zeitintervall (450) kleiner als eine Schwingungsperiode (460) des Anregungsstromes ist.

6. Regelschaltung (202) gemäß Anspruch 5, welche ferner eine Reihenschaltung (302) eines ersten elektrischen Bauteils (304) und eines zweiten elektrischen Bauteils (306) umfasst, wobei der Schwingkreis (208; 300) an eine elektrisch leitende Verbindung (308) zwischen dem ersten elektrischen Bauteil (304) und dem zweiten elektrischen Bauteil (306) gekoppelt ist, sodass der Anregungsstrom durch ein erstes Eingangssignal (316) des ersten elektrischen Bauteils (304) und/oder ein zweites Eingangssignal (318) des zweiten elektrischen Bauteils (306) bewirkt wird.

7. Regelschaltung (202) gemäß Anspruch 6, ferner umfassend wenigstens eine erste Spannungsquelle, welche dazu ausgebildet ist, das erste Eingangssignal (316) derart zu erzeugen, dass das erste Eingangssignal (316) wechselweise in dem Zeitintervall (450) einen steigenden oder fallenden Verlauf und in einem weiteren Zeitintervall (470) einen konstanten Verlauf aufweist.

8. Regelschaltung (202) gemäß Anspruch 7, wobei ein Verhältnis des Zeitintervalls (450) zu dem weiteren Zeitintervall (470) kleiner als 1 ist.

9. Regelschaltung (202) gemäß einem der Ansprüche 7 oder 8, welche dazu ausgebildet ist, ein Verhältnis des Zeitintervalls (450) zu dem weiteren Zeitintervall (470) zu regeln.

10. Regelschaltung (202) gemäß Anspruch 9, welche dazu ausgebildet ist, durch ein Vergrößern des Verhältnisses des Zeitintervalls (450) zu dem weiteren Zeitintervalls (470) ein Verringern der Resonanzfrequenz oder durch ein Verringern des Verhältnisses des Zeitintervalls (450) zu dem weiteren Zeitintervall (470) ein Vergrößern der Resonanzfrequenz zu bewirken.

11. Regelschaltung (202) gemäß einem der Ansprüche 7 bis 10, ferner umfassend wenigstens eine zweite Spannungsquelle, welche dazu ausgebildet ist, das zweite Eingangssignal (318) derart zu erzeugen, dass das zweite Eingangssignal (318) wechselweise in dem Zeitintervall (450) einen steigenden oder fallenden Verlauf und in dem weiteren Zeitintervall (470) einen konstanten Verlauf aufweist, wobei der steigende Verlauf des ersten Eingangssignals (316) mit dem fallenden Verlauf des zweiten Eingangssignals (318) oder der steigende Verlauf des zweiten Eingangssignals (318) mit dem fallenden Verlauf des ersten Eingangssignals (316) koinzidieren.

12. Regelschaltung (202) gemäß einem der Ansprüche 6 bis 11, wobei das erste elektrische Bauteil (304) ein erster Verstärker, das zweite elektrische Bauteil (306) ein zweiter Verstärker, das erste Eingangssignal (316) eine erste Eingangsspannung und das zweite Eingangssignal (318) eine zweite Eingangs Spannung sind.

13. Regelschaltung (202) gemäß einem der Ansprüche 6 bis 11, wobei das erste elektrische Bauteil (304) ein erster Transistor, das zweite elektrische Bauteil (306) ein zweiter Transistor, das erste Eingangssignal (316) eine erste Steuerspannung und das zweite Eingangssignal (318) eine zweite Steuerspannung sind.

14. Regelschaltung (202) gemäß einem der Ansprüche 6 bis 13, welche ferner eine Reihenschaltung eines dritten elektrischen Bauteils und eines vierten elektrischen Bauteils umfasst, wobei der Schwingkreis (208; 300) an einen Brückenzweig zwischen einer elektrisch leitenden Verbindung zwischen dem ersten elektrischen Bauteil (304) und dem zweiten elektrischen Bauteil (306) und einer elektrisch leitenden Verbindung zwischen dem dritten elektrischen Bauteil und dem vierten elektrischen Bauteil koppelt, sodass der Anregungsström durch das erste Eingangssignal (316), das zweite Eingangssignal (318), ein drittes Eingangssignal des dritten elektrischen Bauteils und/oder ein viertes Eingangssignal des vierten elektrischen Bauteils bewirkt wird.

15. Basisstation (204) mit einer Regelschaltung (202) gemäß einem der vorangegangenen Ansprüche, wobei der Schwingkreis (208; 300) dazu ausgebildet ist, ein Rückkopplungssignal mit einer Modulationseigenschaft zu empfangen, welche Informationen über eine auf den Empfängerschwingkreis (212) des Empfängers (206) übertragene Energie umfasst.

**16.** System, umfassend einen Empfänger (206) und eine Basisstation (204) gemäß Anspruch 16, wobei der Empfänger (206) dazu ausgebildet ist, das Energiesignal zu empfangen und das Rückkopplungssignal zu senden.

**Claims**

**1.** Closed-loop control circuit (202) for a base station (204) for transmitting energy to a receiver (206) by means of an electrical resonant circuit (208; 300), comprising:

an electrical resonant circuit (208; 300);
an evaluation device (210) which is designed to compare energy transmitted to a receiver resonant circuit (212) of the receiver (206) with a target energy value,
wherein the closed-loop control circuit (202) is designed to cause a changed energy input into the receiver resonant circuit (212) of the receiver (206) by changing a resonant frequency of the resonant circuit (208; 300) on the basis of a result of the comparison,
wherein the evaluation device (210) is designed to determine the transmitted energy on the basis of a modulation property of a feedback signal that couples to the electrical resonant circuit (208; 300),
wherein the closed-loop control circuit (202) is designed to at least partially counteract a change in the coupling of the resonant circuit (208; 300) to the receiver resonant circuit (212) by changing the resonant frequency of the resonant circuit (208; 300),
wherein the closed-loop control circuit (202) is designed to move the resonant frequency of the resonant circuit (208; 300) away from a natural frequency of the receiver resonant circuit (212) if the transmitted energy is greater than the target energy value,
**characterized in that** the closed-loop control circuit comprises a resistive element which is coupled to the resonant circuit (208; 300), has a temporally variable resistivity (314) and comprises an open-loop control connection for receiving an open-loop control signal for changing the resistivity (314).

**2.** Closed-loop control circuit (202) according to Claim 1, which is designed to bring the resonant frequency of the resonant circuit (208; 300) closer to a natural frequency of the receiver resonant circuit (212) if the transmitted energy is less than the target energy value.

**3.** Closed-loop control circuit (202) according to one of Claims 1 to 3, which is designed to change the resonant frequency by changing an electrical resistance (314) in the resonant circuit (208; 300).

**4.** Closed-loop control circuit (202) according to Claim 3, which is designed to change the electrical resistance (314) by varying an excitation current, wherein the electrical resistance (314) is an effective resistance of the resonant circuit (208; 300).

**5.** Closed-loop control circuit (202) according to Claim 4, which is designed to change the electrical resistance (314) within a predetermined interval of time (450), wherein the interval of time (450) is shorter than an oscillation period (460) of the excitation current.

**6.** Closed-loop control circuit (202) according to Claim 5, which also comprises a series circuit (302) comprising a first electrical component (304) and a second electrical component (306), wherein the resonant circuit (208; 300) is coupled to an electrically conductive connection (308) between the first electrical component (304) and the second electrical component (306), with the result that the excitation current is caused by a first input signal (316) from the first electrical component (304) and/or a second input signal (318) from the second electrical component (306).

**7.** Closed-loop control circuit (202) according to Claim 6, also comprising at least one first voltage source which is designed to generate the first input signal (316) in such a manner that the first input signal (316) alternately has a rising or falling profile in the interval of time (450) and a constant profile in a further interval of time (470).

**8.** Closed-loop control circuit (202) according to Claim 7, wherein a ratio of the interval of time (450) to the further interval of time (470) is less than 1.

**9.** Closed-loop control circuit (202) according to either of Claims 7 and 8, which is designed to subject a ratio of the interval of time (450) to the further interval of time (470) to closed-loop control.

**10.** Closed-loop control circuit (202) according to Claim 9, which is designed to reduce the resonant frequency by increasing the ratio of the interval of time (450) to the further interval of time (470) or to increase the resonant frequency by reducing the ratio of the interval of time (450) to the further interval of time (470).

11. Closed-loop control circuit (202) according to one of Claims 7 to 10, also comprising at least one second voltage source which is designed to generate the second input signal (318) in such a manner that the second input signal (318) alternately has a rising or falling profile in the interval of time (450) and a constant profile in the further interval of time (470), wherein the rising profile of the first input signal (316) coincides with the falling profile of the second input signal (318) or the rising profile of the second input signal (318) coincides with the falling profile of the first input signal (316).

12. Closed-loop control circuit (202) according to one of Claims 6 to 11, wherein the first electrical component (304) is a first amplifier, the second electrical component (306) is a second amplifier, the first input signal (316) is a first input voltage and the second input signal (318) is a second input voltage.

13. Closed-loop control circuit (202) according to one of Claims 6 to 11, wherein the first electrical component (304) is a first transistor, the second electrical component (306) is a second transistor, the first input signal (316) is a first open-loop control voltage and the second input signal (318) is a second open-loop control voltage.

14. Closed-loop control circuit (202) according to one of Claims 6 to 13, which also comprises a series circuit comprising a third electrical component and a fourth electrical component, wherein the resonant circuit (208; 300) couples to a bridge arm between an electrically conductive connection between the first electrical component (304) and the second electrical component (306) and an electrically conductive connection between the third electrical component and the fourth electrical component, with the result that the excitation current is caused by the first input signal (316), the second input signal (318), a third input signal from the third electrical component and/or a fourth input signal from the fourth electrical component.

15. Base station (204) having a closed-loop control circuit (202) according to one of the preceding claims, wherein the resonant circuit (208; 300) is designed to receive a feedback signal with a modulation property comprising information about energy transmitted to the receiver resonant circuit (212) of the receiver (206).

16. System comprising a receiver (206) and a base station (204) according to Claim 16, wherein the receiver (206) is designed to receive the energy signal and to transmit the feedback signal.

**Revendications**

1. Circuit de régulation (202) pour une station de base (204) destiné à la transmission d'une énergie à un récepteur (206) au moyen d'un circuit électrique LC (208 ; 300), comprenant :

   un circuit électrique LC (208 ; 300) ;
   un dispositif d'évaluation (210) qui est réalisé pour comparer une énergie transmise sur un circuit LC de réception (212) du récepteur (206) avec une valeur d'énergie de consigne,
   dans lequel le circuit de régulation (202) est réalisé pour provoquer un apport en énergie modifié dans le circuit LC de réception (212) du récepteur (206) par une modification d'une fréquence de résonance du circuit LC (208 ; 300), basée sur un résultat de la comparaison,
   dans lequel le dispositif d'évaluation (210) est réalisé pour déterminer l'énergie transmise sur la base d'une propriété de modulation d'un signal de rétroaction couplé au circuit électrique LC (208 ; 300),
   dans lequel le circuit de régulation (202) est réalisé pour contrer au moins partiellement une modification d'un couplage du circuit LC (208 ; 300) au circuit LC de réception (212) par la modification de la fréquence de résonance du circuit LC (208 ; 300),
   dans lequel le circuit de régulation (202) est réalisé pour retirer la fréquence de résonance du circuit LC (208 ; 300) d'une fréquence naturelle du circuit LC de réception (212) si l'énergie transmise est supérieure à la valeur d'énergie de consigne,
   **caractérisé en ce que** le circuit de régulation comprend un élément résistif à résistivité variable dans le temps (314), couplé au circuit LC (208 ; 300), qui comprend une borne de commande pour recevoir un signal de commande servant à modifier la résistivité (314) .

2. Circuit de régulation (202) selon la revendication 1, qui est réalisé pour rapprocher la fréquence de résonance du circuit LC (208 ; 300) d'une fréquence naturelle du circuit LC de réception (212) si l'énergie transmise est inférieure à la valeur d'énergie de consigne.

3. Circuit de régulation (202) selon l'une quelconque des revendications 1 à 3, qui est réalisé pour provoquer la modification de la fréquence de résonance par une modification de la résistance électrique (314) dans le circuit LC (208 ; 300).

4. Circuit de régulation (202) selon la revendication 3, qui est réalisé pour provoquer la modification de la résistance électrique (314) par une variation d'un courant d'excitation, dans lequel la résistance électrique (314) est une résistance effective du circuit LC (208 ; 300).

5. Circuit de régulation (202) selon la revendication 4, qui est réalisé pour modifier la résistance électrique (314) à l'intérieur d'un intervalle de temps prédéterminé (450), dans lequel l'intervalle de temps (450) est inférieur à une période (460) du courant d'excitation.

6. Circuit de régulation (202) selon la revendication 5, comprenant en outre un montage en série (302) d'un premier composant électrique (304) et d'un deuxième composant électrique (306), dans lequel le circuit LC (208 ; 300) est couplé à une connexion électriquement conductrice (308) entre le premier composant électrique (304) et le deuxième composant électrique (306) de sorte que le courant d'excitation est provoqué par un premier signal d'entrée (316) du premier composant électrique (304) et/ou un deuxième signal d'entrée (318) du deuxième composant électrique (306).

7. Circuit de régulation (202) selon la revendication 6, comprenant en outre au moins une première source de tension qui est réalisée pour générer le premier signal d'entrée (316) de telle sorte que le premier signal d'entrée (316) présente en alternance dans l'intervalle de temps (450) une allure montante ou descendante et présente dans un intervalle de temps supplémentaire (470) une allure constante.

8. Circuit de régulation (202) selon la revendication 7, dans lequel un rapport entre l'intervalle de temps (450) et l'intervalle de temps supplémentaire (470) est inférieur à 1.

9. Circuit de régulation (202) selon l'une quelconque des revendications 7 ou 8, qui est réalisé pour réguler un rapport entre l'intervalle de temps (450) et l'intervalle de temps supplémentaire (470).

10. Circuit de régulation (202) selon la revendication 9, qui est réalisé pour provoquer une diminution de la fréquence de résonance par une augmentation du rapport entre l'intervalle de temps (450) et l'intervalle de temps supplémentaire (470), et pour provoquer une augmentation de la fréquence de résonance par une diminution du rapport entre l'intervalle de temps (450) et l'intervalle de temps supplémentaire (470).

11. Circuit de régulation (202) selon l'une quelconque des revendications 7 à 10, comprenant en outre au moins une deuxième source de tension qui est réalisée pour générer le deuxième signal d'entrée (318) de telle sorte que le deuxième signal d'entrée (318) présente dans l'intervalle de temps (450) en alternance une allure montante ou descendante et dans l'intervalle de temps supplémentaire (470) une allure constante, dans lequel l'allure montante du premier signal d'entrée (316) coïncide avec l'allure descendante du deuxième signal d'entrée (318), ou l'allure montante du deuxième signal d'entrée (318) coïncide avec l'allure descendante du premier signal d'entrée (316).

12. Circuit de régulation (202) selon l'une quelconque des revendications 6 à 11, dans lequel le premier composant électrique (304) est un premier amplificateur, le deuxième composant électrique (306) est un deuxième amplificateur, le premier signal d'entrée (316) est une première tension d'entrée et le deuxième signal d'entrée (318) est une deuxième tension d'entrée.

13. Circuit de régulation (202) selon l'une quelconque des revendications 6 à 11, dans lequel le premier composant électrique (304) est un premier transistor, le deuxième composant électrique (306) est un deuxième transistor, le premier signal d'entrée (316) est une première tension de commande et le deuxième signal d'entrée (318) est une deuxième tension de commande.

14. Circuit de régulation (202) selon l'une quelconque des revendications 6 à 13, qui comprend en outre un montage en série d'un troisième composant électrique et d'un quatrième composant électrique, dans lequel le circuit LC (208 ; 300) est couplé à une branche de pont entre une connexion électriquement conductrice entre le premier composant électrique (304) et le deuxième composant électrique (306) et une connexion électriquement conductrice entre le troisième composant électrique et le quatrième composant électrique de sorte que le courant d'excitation est provoqué par le premier signal d'entrée (316), le deuxième signal d'entrée (318), un troisième signal d'entrée du troisième composant électrique et/ou un quatrième signal d'entrée du quatrième composant électrique.

15. Station de base (204), comprenant un circuit de régulation (202) selon l'une quelconque des revendications précédentes, dans laquelle le circuit LC (208 ; 300) est réalisé pour recevoir un signal de rétroaction ayant une propriété de modulation qui comprend des informations concernant une énergie transmise au circuit LC de réception (212) du récepteur (206).

16. Système, comprenant un récepteur (206) et une station de base (204) selon la revendication 16, dans lequel le récepteur (206) est réalisé pour recevoir le signal d'énergie et pour envoyer le signal de rétroaction.

Fig. 1

Fig. 2

Fig. 3

Fig. 4

500

| Ermitteln | ~ 510 |

| Vergleichen | ~ 520 |

| Bewirken | ~ 530 |

Fig. 5

Fig. 6

Fig. 7

Fig. 8

Fig. 9

Fig. 10a

Fig. 10b

1100

| Bestimmen | ～1110 |
| Ermitteln | ～1120 |

Fig. 11

**EP 3 226 970 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2009051539 A1
- WO 2009091267 A2
- US 2014339910 A1
- US 2012212074 A1